# EUROPEAN PATENT APPLICATION

(11) **EP 3 944 850 A1**
(43) Date of publication of application: **02.02.2022**
(21) Application number: 20305883.9
(22) Date of filing: 31.07.2020
(51) Int. Cl.: A61J 1/20, A61M 5/162, A61M 39/10

(54) **MULTI-PART ADAPTER ASSEMBLY FOR BULK MEDICAL FLUID CONTAINER**

(71) Applicant: Guerbet, 93420 Villepinte (FR)
(72) Inventor: ALLARD, Ludovic, 69390 MILLERY (FR)
(74) Representative: Regimbeau

(57) **Abstract**

A medical container assembly is disclosed having an adapter assembly that fluidly connects with a medical container having a volume of medical fluid to accommodate multiple injections. The adapter assembly includes a spike assembly (300) that fluidly connects with the medical container, along with a spike interface cover (200) that detachably connects with the spike assembly (300). Features are incorporated by the spike assembly (300) to reduce the potential that the spike assembly (300) will be used with multiple medical containers. The spike interface cover (200) is detachably connected with the spike assembly (300) and includes features that allows the spike interface cover (200) to be used with a spike assembly (300) installed on one container, and to thereafter allow this same spike interface cover (200) to be used with another spike assembly (300) installed on another container.

## Description

### FIELD

The present invention generally relates to injection of medical fluids and, more particularly, to the use of a bulk medical fluid source for the injection of multiple patients.

### BACKGROUND

The injection of iodinated contrast agent is required in 70% of CT scan diagnosis cases. This injection in 70% of cases is performed using an automated contrast agent injector. Injection tubing is required to connect the automated injector to the patient.

For economic and ecological reasons (less use of plastic), the multi-patient practice is steadily gaining market share. Within 5 to 10 years, it is possible to imagine that multi-patient use will account for more than 75% of the world market for contrast agent injection. In the so-called multi-patient practice, the tubing for the injector is provided with two very distinct parts: the patient set and the day set. The patient set is typically used to limit risks of cross contamination between consecutive patients and thus protects the day set. Once installed and primed the day set stays connected on the power injector for several patient cases. This day set comprises a filling line which is connected to the primary container.

The connection to the primary container may be made either with a male Luer-lock, to connect to a ScanBag^{®} (Guerbet solution), or more generally with a spike to connect to a bottle. More than 95% of primary containers of contrast agent or saline are bottles, usually made of glass or flexible plastic. The "common" part is the capping area, which is systematically equipped with a soft area, the septum. The septum is a piece of soft plastic material, usually around 70 or 80 ShA, with the feature of being perforable by a pointed shape. The spike is connected to the primary container, by perforation of the septum, which then gives access to the liquid contained in the container.

In multi-patient use, the practice encourages the use of tubing for 12 hours, even 24 hours. In 24 hours, nearly 80 patients can be injected using the same tubing. If the average injection of contrast agent is 100 mL, considering the largest 500 mL container of contrast agent or saline, between 16 and 32 primary containers can easily be used in 24 hours, depending on the volume of saline solution injected and the volume of the saline solution container.

The spike is typically a plastic needle, most often connected to a drip chamber and its main function is to perforate the septum of the primary container and thus allow access to the contrast agent and allow it to flow into the filling tubing. Between two bottles, to switch from an empty bottle to a full one, the spike is pulled from the first empty bottle, and is left in the open air before being inserted into a new bottle. The needle area of the spike, which is quite large, may at this time come into contact with an inadvertent contaminated area or may be contaminated by the ambient air, since CT suites are not clean rooms and examination areas are not sterile.

With the deposition of contamination on the needle, when the next bottle is perforated, the contamination can get into the bottle and contaminate the 500 mL of contrast agent. This contamination will thus be able to spread from person to person, as successive injections are made, to the filling tubing, then to the syringe, then to the injection tubing and to the patient.

In order to be able to perforate different types of septums (because of the different types of bottles) the spike needle is typically quite large in diameter (roughly 5 to 6 mm) and quite long (roughly 4 to 6 cm). The contact surface of spike needle (that is exposed to the ambient environment) is therefore quite large, roughly 11.5 cm². Added to a sometimes long interconnection time between two bottles, to a poorly disinfected bottle septum, or to a handling error (falling to the ground for example), the risk of contamination can be quite high.

### SUMMARY

A medical container assembly is presented herein. Both the configuration of individual components of this medical container assembly, along with the manner of connecting one or more individual components of this medical container assembly, are within the scope of this Summary. The terms "proximal" and "distal" are used herein in relation to a patient being injected using the medical container assembly, with the "proximal" being closer to the patient than the "distal."

A medical container may be of any appropriate configuration and may include any appropriate medical fluid (e.g., contrast media). The medical container may include at least one outlet that is closed by a septum, seal, or the like. A medical container adapter assembly may include a spike assembly that accesses the interior of the medical container through the septum, along with a spike interface cover that may detachably engage the spike assembly, both prior to installing the spike assembly on the medical container and after the spike assembly has been installed on the medical container, for instance to allow for removal of the spike interface cover from the spike assembly for subsequent use of the same spike interface cover with another medical container and another spike assembly. That is, the spike assembly may be configured as a single-use component (e.g., for use with a single medical container), while the spike interface cover may be configured as a multi-use component (e.g., usable with a first spike assembly on a first medical container when the spike interface cover is detachably engaged with the first spike assembly; usable with a second spike assembly (another spike assembly) installed on a second medical container (another medical container) when the spike interface cover is detachably engaged with the second spike assembly).

The spike assembly may include a container perforator that defines a distal end of the spike assembly - the end that engages a septum of a medical container. One or more air passages may extend from an exterior of the container perforator and through an interior of the spike assembly proceeding along a length dimension of the adapter assembly. Similarly, one or more liquid passages may extend from an exterior of the container perforator and through the interior of the spike assembly proceeding along the length dimension of the adapter assembly. One or more embodiments have each air passage intersecting the exterior of the container perforator at a location that is closer to a distal tip of the container perforator (measured along the length dimension of the adapter assembly), compared to where each liquid passage intersects the exterior of the container perforator. A plurality of elongate liquid inlet channels may be disposed in circumferentially-spaced relation on an exterior of the container perforator and may include at least one liquid inlet port on a proximal end of the corresponding elongate liquid inlet channel (e.g., to direct liquid to one or more interior liquid passages of the spike assembly).

One or more features may be incorporated by the container perforator that address the interaction of the container perforator with a septum of a medical container (e.g., in relation to installation of the spike assembly; in relation to at least impeding use of the spike assembly with two or more medical containers). A frusto-conical exterior section of the container perforator may extend from a distal tip of the container perforator (e.g., convexly-shaped, defined by a small radius, or both). An exterior transition section of the container perforator may extend proximally relative to the frusto-conical exterior section (including where the exterior transition section extends approximately from the frusto-conical exterior section). This exterior transition section of the container perforator may be concave proceeding along a length dimension of the container perforator, and which may facilitate installation of the container perforator in a septum of a medical container. The air passage may intersect with the exterior of the container perforator at least within this exterior transition section.

A plurality of elongate channels may be disposed on the exterior of the container perforator, may be disposed in spaced relation to one another, and may be concave relative to the exterior of the container perforator. A width of each of the elongate channels may progressively decrease proceeding away from a distal tip of the container perforator. These elongate channels may reduce the surface area of the container perforator that interfaces with a septum of a medical container during the initial portion of the installation of the spike assembly on the medical container, and thereby may reduce the amount of force required to install the spike assembly. The container perforator may be installed on a distal end of a perforator extension that extends proximally from the container perforator, and this perforator extension may be engaged by a septum of a medical container when the spike assembly is in an installed configuration (e.g., to provide a suitable seal between the spike assembly and the septum of the medical container to reduce the potential of leakage of medical fluid from the medical container after installation of the spike assembly).

A proximal end of the container perforator may include a plurality of retention members or tabs, including three or more retention members that are spaced about a circumference of the container perforator. Each retention member may extend away from a central reference axis of the container perforator proceeding in a direction that is away from a distal tip of the container perforator and to a free end of the corresponding retention member. At least a lower or proximal section of each retention member may curve away from the central reference axis proceeding in the direction of their corresponding free end. A space may be disposed adjacent to and radially inward of the lower or proximal section of each retention member. This allows each retention member to elastically deflect radially inwardly (reducing the corresponding outer diameter of the container perforator) as the container perforator is being directed through a septum of a medical container (e.g., reducing the required installation force), and furthermore allows each such retention member to move back toward their original position (e.g., to an undeformed state; increasing the corresponding outer diameter of the container perforator) after completely entering the interior of the medical container (e.g., when the retention members "clear" an interior surface of the septum) and which should thereafter impede removal of the container perforator from the medical container (e.g., as the "flared out" retention members should at some time engage the interior surface of the septum).

At least the container perforator may be configured to separate from a remainder of the spike assembly upon exposure to a first force. The spike assembly may be configured such that this first force would be insufficient to withdraw the container perforator from a septum of a medical container when the spike assembly is in an installed configuration. Upon exposure to this first force, the container perforator should remain installed on the medical container such that the spike assembly may not be re-used with another medical container, and which should reduce the potential for patient cross-contamination.

The spike assembly may be characterized as including a base having an upper surface, along with a perforator that extends distally from this upper surface. The perforator may include the above-noted container perforator, alone or in combination with the above-noted perforator extension. The container perforator, the perforator extension, or both may each contain at least a portion of one or more liquid passages for the spike assembly and one or more air passages for the spike assembly. The upper surface of the base may be flat or planar, and the perforator may extend orthogonally in the distal dimension from this upper surface. One or more embodiments entail an outer diameter of the base being larger than an outer diameter of the perforator (e.g., a maximum outer diameter of the perforator).

The spike assembly may include an interior air chamber and a separate interior liquid chamber. Each liquid passage of the perforator may extend from an exterior of the perforator to the liquid chamber. Similarly, each air passage of the perforator may extend from an exterior of the perforator to the air chamber. A proximal end of the spike assembly may include an interface that is formed from a material having a reduced hardness compared to at least an adjacent portion of the spike assembly. This allows a corresponding perforator of the spike interface cover to extend through a corresponding portion of the interface to fluidly connect with the corresponding liquid chamber or air chamber of the spike assembly.

The spike interface cover may be detachably connected to and detachably removed from the spike assembly, both prior to installing the spike assembly on a medical container and after the spike assembly has been installed on a medical container (and all without damaging the spike interface cover such that the spike interface cover may be used with another spike assembly/medical container). A distal end of the spike interface cover may include a receptacle for receiving a proximal portion of the spike assembly. This receptacle may be defined at least by a plurality of upper wall segments that extend distally from a distal base surface (e.g., of a flat or planar configuration) of the spike interface cover.

A plurality of movable clips may extend distally beyond the distal base surface of the spike interface cover (and that may further define at last part of the above-noted noted receptacle). Each such clip may move between an open position (e.g., to allow the spike interface cover to be detached from the spike assembly, for instance by a user engaging and directing an actuating surface of each clip in an inward direction) and a closed or latching position where a head of each clip engages the spike assembly in a manner that detachably connects the spike interface cover with the spike assembly (e.g., engagement of the clips of the spike interface cover with the spike assembly may limit or preclude movement of the spike interface cover away from the spike assembly along an axial path to separate these components). The noted motion of the clips may be a pivotal or pivotal-like motion (e.g., via a living hinge between a given clip and a corresponding portion of the spike interface cover). Each clip may be biased (e.g., elastically) toward its closed or latching position. The spike assembly, the spike interface cover, or both may include features to require the spike interface cover to be installed on the spike assembly in only a single orientation, for instance by having the head of one clip of the spike interface cover being wider than the head of another clip of the spike interface cover, and by having the spike assembly include channels of a corresponding width on an outer perimeter of the spike assembly.

The spike interface cover may include a perforator that extends into the air chamber of the spike assembly (e.g., by perforating/extending through a corresponding portion of the interface on the proximal end of the spike assembly), and another perforator that extends into the liquid chamber of the spike assembly (e.g., by perforating/extending through a corresponding portion of the interface on the proximal end of the spike assembly). These perforators may extend distally from the noted distal base surface of the spike interface cover. A distal end of these perforators may be proximately offset from the distal end of (e.g., recessed from) the upper wall segments and/or the heads of the noted clips, which should reduce the potential for contamination of the spike interface cover perforators prior to installing the spike interface cover on the spike assembly.

One or more air passages may extend through the corresponding perforator of the spike interface cover (the perforator that extends into the air chamber of the spike assembly). External air that is directed into one or more air passages of the spike interface cover may pass through a one or more filters prior to entering the air chamber of the spike assembly. One or liquid passages may extend through the corresponding perforator of the spike interface cover (the perforator that extends into the liquid chamber of the spike assembly). Each liquid passage of the spike interface cover may extend to a container that may be interconnected with/extend from a proximal end of the spike interface cover (e.g., a drip chamber). This container may include a medical conduit interface that is configured to connect a medical conduit of any appropriate type to this container.

Any feature of any of the various aspects of the present invention that is intended to be limited to a "singular" context or the like will be clearly set forth herein by terms such as "only," "single," "limited to," or the like. Merely introducing a feature in this disclosure in accordance with commonly accepted antecedent basis practice does not limit the corresponding feature to the singular (e.g., indicating that a container perforator includes "a liquid passage" alone does not mean that the container perforator includes only a single liquid passage). Moreover, any failure herein to use phrases such as "at least one" also does not limit the corresponding feature to the singular (e.g., indicating that a container perforator includes "a liquid passage" alone does not mean that the container perforator includes only a single liquid passage). Use of the phrase "at least generally" or the like in relation to a particular feature encompasses the corresponding characteristic and insubstantial variations thereof (e.g., indicating that a base surface is at least generally flat encompasses the base surface actually being flat). Finally, a reference of a feature in conjunction with the phrase "in one embodiment" does not limit the use of the feature to a single embodiment.

Various aspects of the present invention are also addressed by the following paragraphs and in the noted combinations:
1. A medical fluid container adapter assembly, comprising:
   a first connector part comprising a container perforator, a first liquid passage and a first air passage, wherein said container perforator comprises a distal tip and an exterior, and wherein said first connector part is connectable to a medical fluid container and is a single use connector part, wherein the first connector is a single use connector part; and
   a multi-use assembly detachably connected to said first connector part, wherein said multi-use assembly comprises a medical conduit interface and a second connector part comprising a second liquid passage and a second air passage, and wherein said first liquid passage and said first air passage of said first connector part are fluidly connected with said second liquid passage and said second air passage, respectively, of said second connector part.
2. The medical fluid container adapter assembly of paragraph 1, wherein said first connector part comprises a spike assembly.
3. The medical fluid container adapter assembly of any of paragraphs 1-2, wherein said first connector part further comprises a first body in turn comprising a first base distal surface, said container perforator protrudes distally relative to said first base distal surface, and said container perforator comprises said first liquid passage and said first air passage.
4. The medical fluid container adapter assembly of paragraph 3, wherein a separation force is less than a removal force, said separation force is a minimum force required to separate said container perforator from said first body, said removal force is a minimum force required to remove said container perforator from the medical fluid container when said medical container adapter assembly is in an installed configuration, and said separation force is less than said removal force.
5. The medical fluid container adapter assembly of paragraph 3, wherein said first connector part is configured such that said container perforator will separate from said first body when exposed to a first force, and said first force is insufficient to remove said container perforator from the medical fluid container when said medical fluid container adapter assembly is in an installed configuration and with said container perforator remaining connected with said first body.
6. The medical fluid container adapter assembly of any of paragraphs 3-5, wherein said first body further comprises a base and a perforator extension, said base comprises said first base distal surface, said perforator extension extends distally from said first base distal surface and comprises said first liquid passage and said first air passage, and said container perforator is interconnected with said perforator extension.
7. The medical fluid container adapter assembly of any of paragraphs 3-6, wherein said first body comprises a liquid chamber fluidly connected with said first liquid passage and an air chamber fluidly connected with said first air passage, wherein said second liquid passage of said second connector part is fluidly connected with said liquid chamber and said second air passage of said second connector part is fluidly connected with said air chamber.
8. The medical fluid container adapter assembly of any of paragraphs 1-7, wherein said second air passage of said second connector part comprises a filter.
9. The medical fluid container adapter assembly of any of paragraphs 1-8, wherein said second air passage of said second connector part terminates on an exterior of said second connector part.
10. The medical fluid container adapter assembly of any of paragraphs 1-9, wherein said second connector part comprises a distal end that comprises a receptacle, wherein said first body of said first connector part extends proximally within said receptacle.
11. The medical fluid container adapter assembly of any of paragraphs 3-6, 8, and 9, wherein said first body comprises a proximal end spaced from said first base distal surface of said first body, said first connector part comprises a first interface disposed on said proximal end of said first body that interfaces with said second connector part, and said first interface is of a first hardness and said first body is of a second hardness that is greater than said first hardness.
12. The medical fluid container adapter assembly of paragraph 11, wherein said first interface of said first connector part comprises a first perforable section in a flow path to said first liquid passage and a second perforable section in a flow path to said first air passage.
13. The medical fluid container adapter assembly of paragraph 12, wherein said second connector part comprises a second base distal surface, a perforator for said second liquid passage, and a perforator for said second air passage, wherein said perforator for said second liquid passage protrudes distally from said second base distal surface, is fluidly connected with said second liquid passage, and extends through said first perforable section of said first interface for said first connector part, and wherein said perforator for said second air passage protrudes from said second base distal surface, is fluidly connected with said second air passage, and extends through said second perforable section of said first interface for said first connector part.
14. The medical fluid container adapter assembly of paragraph 13, wherein said first interface is configured to deform when said perforator for said second liquid passage of said second connector part is directed into and through said first perforable section of said first interface for said first connector part, and is further configured to deform when said perforator for said second air passage of said second connector part is directed into and through said second perforable section of said first interface for said first connector part.
15. The medical fluid container adapter assembly of any of paragraphs 13-14, wherein said first interface of said first connector part seals against each of said perforator for said second liquid passage of said second connector part and said perforator for said second air passage of said second connector part.
16. The medical fluid container adapter assembly of any of paragraphs 13-15, wherein said first perforable section and said second perforable section each comprises a plurality of segments configured to separate from one another along a predetermined path.
17. The medical fluid container adapter assembly of any of paragraphs 13-16, wherein said perforator for said second liquid passage comprises said second liquid passage and said perforator for said second air passage comprises said second air passage.
18. The medical fluid container adapter assembly of any of paragraphs 13-17, wherein said first body comprises a liquid chamber fluidly connected with said first liquid passage and an air chamber fluidly connected with said first air passage, wherein said second liquid passage of said second connector part is fluidly connected with said liquid chamber and said second air passage of said second connector part is fluidly connected with said air chamber, wherein said perforator for said second liquid passage extends into said liquid chamber, and wherein said perforator for said second air passage extends into said air chamber.
19. The medical fluid container adapter assembly of any of paragraphs 13-18, wherein said second part further comprises a plurality of upper wall segments that are spaced from one another and that protrude distally from said second base distal surface of said second connector part, wherein said perforator for said second liquid passage and said perforator for said second air passage are located inwardly of an outer perimeter collectively defined by said plurality of upper wall segments.
20. The medical fluid container adapter assembly of paragraph 19, wherein a distal end of each of said perforator for said second liquid passage and said perforator for said second air passage are disposed closer to said second base distal surface than a distal end of each of said plurality of upper wall segments.
21. The medical fluid container adapter assembly of any of paragraphs 19-20, wherein a first and second upper wall segment of said plurality of upper wall segments are movable between open and closed positions to detachably connect said second connector part to said first connector part.
22. The medical fluid container adapter assembly of any of paragraphs 19-21, wherein said plurality of upper wall segments of said second connector part are disposed outwardly of said first connector part.
23. The medical fluid container adapter assembly of any of paragraphs 19-22, wherein an outer diameter of a proximal portion of said first body is greater than an outer diameter of said container perforator, and wherein an outer perimeter of said proximal portion of said first body is at least one of disposed in closely spaced relation to or in contact with said plurality of upper wall segments of said second connector part.
24. The medical fluid container adapter assembly of paragraph 11, wherein said first interface comprises a first region and a second region, and each of said first region and second region comprises a plurality of seal segments that are separated from one another by a thin-walled section.
25. The medical fluid container adapter assembly of paragraph 24, wherein said second connector part comprises a second base distal surface, a perforator for said second liquid passage, and a perforator for said second air passage, wherein said perforator for said second liquid passage protrudes distally from said second base distal surface, is fluidly connected with said second liquid passage, and extends through said first region of said first interface for said first connector part by separating each adjacent pair of seal segments of said first region and with said seal segments of said first region sealing against an outer perimeter of said perforator for said second liquid passage, wherein said perforator for said second air passage protrudes distally from said second base distal surface, is fluidly connected with said second air passage, and extends through said second region of said first interface for said first connector part by separating each adjacent pair of seal segments of said second region and with said seal segments of said second region sealing against an outer perimeter of said perforator for said second liquid passage.
26. The medical fluid container adapter assembly of any of paragraphs 3-25, wherein said container perforator extends along a first reference axis proceeding distally away relative to said first base distal surface of said first connector part.
27. The medical fluid container adapter assembly of paragraph 26, wherein said container perforator comprises at least 3 retention sections that are spaced from one another, that extend away from said first reference axis proceeding in a direction of said first base distal surface of said first connector part, and that have a free end that is spaced from said first base distal surface of said first connector part, wherein an open space is adjacent to and radially inward of each said retention section.
28. The medical fluid container adapter assembly of paragraph 27, wherein a proximal section of each said retention member curves away from said first reference axis proceeding to a corresponding said free end.
29. The medical fluid container adapter assembly of any of paragraphs 26-28, wherein said distal tip of said container perforator is at least one of convex and defined by a radius.
30. The medical fluid container adapter assembly of paragraph 29, wherein said radius of said distal tip is at least one of less than 0.15 mm or less than 0.05 mm.
31. The medical fluid container adapter assembly of any of paragraphs 26-28, wherein said container perforator comprises a frusto-conical section and a transition section, said frusto-conical section is disposed between said distal tip and said transition section, and said transition section is located between said frusto-conical section and said multi-use assembly.
32. The medical fluid container adapter assembly of paragraph 31, wherein said distal tip of said container perforator is at least one of convex and defined by a radius.
33. The medical fluid container adapter assembly of paragraph 32, wherein said radius of said distal tip is at least one of less than 0.15 mm or less than 0.05 mm.
34. The medical fluid container adapter assembly of any of paragraphs 31-33, wherein said transition section is a concave surface on said exterior of said container perforator proceeding from said frusto-conical section toward said multi-use assembly.
35. The medical fluid container adapter assembly of any of paragraphs 31-34, wherein said container perforator further comprises a concave air inlet port on said exterior surface of said container perforator and fluidly connected with said first air passage.
36. The medical fluid container adapter assembly of paragraph 35, wherein said frusto-conical section and said transition section each comprise said air inlet port.
37. The medical fluid container adapter assembly of any of paragraphs 35-36, wherein said container perforator further comprises a plurality of elongate liquid inlet channels that are spaced from one another on said exterior of said container perforator and that are concave, and wherein each said elongate liquid inlet channel is fluidly connected with at least one said first liquid passage.
38. The medical fluid container adapter assembly of paragraph 37, wherein a closest spacing between said distal tip of said container perforator and where said first air passage intersects said exterior of said container perforator is less than a closest spacing between said distal tip and each said elongate liquid inlet channel.
39. The medical fluid container adapter assembly of any of paragraphs 37-38, further comprising a plurality of elongate channels that are spaced from one another on said exterior of said container perforator, that are disposed between each adjacent pair of elongate liquid inlet channels, and that are concave.
40. The medical fluid container adapter assembly of paragraph 39, wherein a width of each elongate channel of said plurality of elongate channels progressively decreases proceeding proximally away from an end closest to said distal tip of said container perforator.
41. The medical fluid container adapter assembly of any of paragraphs 1-35, wherein said container perforator further comprises a plurality of elongate liquid inlet channels that are spaced from one another on said exterior of said container perforator and that are concave, wherein each said elongate liquid inlet channel is fluidly connected with at least one said first liquid passage.
42. The medical fluid container adapter assembly of paragraph 41, further comprising a plurality of elongate channels that are spaced from one another on said exterior of said container perforator, that are disposed between each adjacent pair of elongate liquid include channels, and that are concave.
43. The medical fluid container adapter assembly of paragraph 42, wherein a width of each elongate channel of said plurality of elongate channels progressively decreases proceeding proximally from an end closest to said distal tip of said container perforator.
44. The medical fluid container adapter assembly of any of paragraphs 1-37, further comprising a plurality of elongate channels that are spaced from one another on said exterior of said container perforator and that are concave.
45. The medical fluid container adapter assembly of paragraph 44, wherein a width of each elongate channel of said plurality of elongate channels progressively decreases proceeding proximally away from an end closest to said distal tip of said container perforator.
46. The medical fluid container adapter assembly of any of paragraphs 3-45, wherein said first body comprises first and second catches that are spaced from one another, wherein said second connector part comprises a second body and first and second clips that are movable relative to said second body and that detachably engage said first and second catches, respectively, to detachably connect said first connector part and said second connector part.
47. The medical fluid container adapter assembly of paragraph 46, wherein said first and second catches are disposed on an outer perimeter of said first body of said first connector part.
48. The medical fluid container adapter assembly of paragraph 47, wherein said first body comprises first and second channels disposed on said outer perimeter of said first body of said first connector part, wherein said first and second catches of said second connector part are disposed within said first and second channels, respectively.
49. The medical fluid container adapter assembly of paragraph 48, wherein said first and second channels of said first connector part are of different widths.
50. The medical fluid container adapter assembly of paragraph 49, wherein said first clip of said second connector part is disposable in said first channel of said first connector part but is not disposable in said second channel of said first connector part.
51. The medical fluid container adapter assembly of paragraph 50, wherein said first clip of said first connector part is wider than said second channel of said first connector part.
52. The medical fluid container adapter assembly of any of paragraphs 48-51, wherein said first and second channels of said first connector part are configured to only accommodate a single relative orientation between said first connector part and said second connector part when detachably connected.
53. The medical fluid container adapter assembly of any of paragraphs 46-52, wherein said first catch comprises a first inclined surface and a first end surface, and wherein said second catch comprises a second inclined surface and a second end surface.
54. The medical fluid container adapter assembly of paragraph 53, wherein said first inclined surface flares outwardly proceeding in a direction of said first end surface and said second inclined surface flares outwardly proceeding in a direction of said second end surface.
55. The medical fluid container adapter assembly of any of paragraphs 53-54, wherein said first and second clips of said second connector part engage said first and second end surfaces, respectively, of said first connector part.
56. The medical fluid container adapter assembly of any of paragraphs 53-55, wherein said first and second end surfaces are oriented at least generally orthogonally to a length dimension of said first connector part, and said container perforator protrudes in said length dimension.
57. The medical fluid container adapter assembly of any of paragraphs 46-56, wherein said first and second clips of said second connector part are pivotable relative to said second body of said second connector part.
58. The medical fluid container adapter assembly of any of paragraphs 46-57, wherein said second connector part further comprises a first living hinge between said first clip and said second body, and a second living hinge between said second clip and said second body.
59. The medical fluid container adapter assembly of any of paragraphs 46-58, wherein said second connector part further comprises a first stop that protrudes from said second body and in that is aligned with said first clip, and a second stop that protrudes from said second body and that is aligned with said second clip.
60. The medical fluid container adapter assembly of paragraph 59, wherein said first stop limits an amount of relative motion between said first clip and said second body and said second stop limits an amount of relative motion between said second clip and said second body.
61. The medical fluid container adapter assembly of any of paragraphs 46-60, further comprising a sterility cover disposed over each of said container perforator, a first latching connection between said first clip of said second connector part and said first catch of said first connector part, and a second latching connection between said second clip of said second connector part and said second catch of said first connector part.
62. The medical fluid container adapter assembly of paragraph 61, wherein said sterility cover comprises at least one detent engaged with said container perforator.
63. The medical fluid container adapter assembly of any of paragraphs 61-62, wherein said sterility cover comprises first and second receivers for said first and second clips, respectively, of said second connector part when engaged with said first and second catches, respectively, of said first connector part.
64. The medical fluid container adapter assembly of paragraph 63, wherein said first and second receivers are disposed in opposing relation to one another.
65. The medical fluid container adapter assembly of any of paragraphs 63-64, wherein said first and second receivers each protrude from an adjacent side wall of said sterility cover.
66. The medical fluid container adapter assembly of any of paragraphs 63-65, wherein said first and second receivers each define an interior space that accommodates movement of said first and second clips relative to said second body of said second connector part when said second connector part is being moved relative to said first connector part to detachably connect said first connector part and said second connector part.
67. The medical fluid container adapter assembly of any of paragraphs 61-66, wherein said sterility cover is removable from said first connector part when said first connector part is detachably connected to said second connector part.
68. The medical fluid container adapter assembly of any of paragraphs 61-67, wherein said sterility cover is a blister pack.
69. The medical fluid container adapter assembly of any of paragraphs 3-12 and 26-68, wherein said second connector part comprises a second base distal surface, a perforator for said second liquid passage, and a perforator for said second air passage, said perforator for said second liquid passage protrudes distally from said second base distal surface and is fluidly connected with said second liquid passage, and said perforator for said second air passage protrudes distally from said second base distal surface and is fluidly connected with said second air passage.
70. The medical fluid container adapter assembly of paragraph 69, wherein said second part further comprises a plurality of upper wall segments that are spaced from one another and that protrude distally from said second base distal surface of said second connector part, wherein said perforator for said second liquid passage and said perforator for said second air passage are located inwardly of an outer perimeter collectively defined by said plurality of upper wall segments.
71. The medical fluid container adapter assembly of paragraph 70, wherein a distal end of each of said perforator for said second liquid passage and said perforator for said second air passage are disposed closer to said second base distal surface than a distal end of each of said plurality of upper wall segments.
72. The medical fluid container adapter assembly of any of paragraphs 70-71, wherein a first and second upper wall segment of said plurality of upper wall segments are movable between open and closed positions to detachably connect said second connector part to said first connector part.
73. The medical fluid container adapter assembly of any of paragraphs 70-72, wherein said plurality of upper wall segments of said second connector part are disposed outwardly of said first connector part.
74. The medical fluid container adapter assembly of any of paragraphs 70-73, wherein an outer diameter of a proximal portion of said first body is greater than an outer diameter of said container perforator, and wherein an outer perimeter of said proximal portion of said first body is at least one of disposed in closely spaced relation to or in contact with said plurality of upper wall segments.
75. The medical fluid container adapter assembly of any of paragraphs 1-74, wherein said multi-use assembly further comprises a drip chamber extending from said second connector part, wherein said second connector part is disposed between said first connector part and said drip chamber.
76. The medical fluid container adapter assembly of paragraph 75, wherein said medical conduit interface extends from said drip chamber, and wherein said drip chamber is disposed between said second connector part and said medical conduit interface.
77. The medical fluid container adapter assembly of any of paragraphs 1-76, wherein said multi-use assembly further comprises a medical conduit extending from said medical conduit interface.
78. A medical container assembly comprising a medical fluid container and The medical fluid container adapter assembly of any of paragraphs 1-77, wherein said medical fluid container comprises a container seal, and wherein said container perforator of said first connector part extends through said container seal.
79. A spike assembly for fluidly accessing a medical fluid container, said spike assembly comprising an elongate spike that in turn comprises:
   a central reference axis that coincides with a length dimension of said elongate spike;
   a distal end and a proximal end that are spaced from one another along a length dimension of said elongate spike;
   a first liquid passage that intersects an exterior of said spike and that proceeds along said length dimension on an interior of said elongate spike;
   a first air passage that intersects said exterior of said elongate spike and that proceeds along said length dimension on said interior of said elongate spike;
   wherein said exterior of said elongate spike comprises a frusto-conical section and a transition section, said frusto-conical section being disposed between said distal end and said transition section, said transition section being located between said frusto-conical section and said proximal end, and said transition section being a concave surface on said exterior of said spike proceeding from said frusto-conical section in a direction of said proximal end.
80. The spike assembly of paragraph 79, wherein said distal end of said elongate spike is at least one of convex and defined by a radius.
81. The spike assembly of paragraph 80, wherein said radius of said distal end is at least one of less than 0.15 mm or less than 0.05 mm.
82. The spike assembly of any of paragraphs 79-81, wherein said first air passage intersects said transition section on said exterior.
83. The spike assembly of any of paragraphs 79-81, wherein said first air passage intersects each of said frusto-conical section said transition section on said exterior.
84. The spike assembly of any of paragraphs 79-83, wherein a distal end section of said spike assembly comprises a container perforator, wherein a distal tip of said container perforator comprises said distal end of said elongate spike.
85. The spike assembly paragraph 84, wherein said container perforator further comprises a plurality of elongate liquid inlet channels that are spaced from one another on said exterior of said container perforator and that are concave, and wherein each said elongate liquid inlet channel is fluidly connected with at least one said first liquid passage.
86. The spike assembly of paragraph 85, wherein a closest spacing between said distal tip of said container perforator and where said first air passage intersects said exterior of said container perforator is less than a closest spacing between said distal tip of said container perforator and each said elongate liquid inlet channel.
87. The spike assembly of any of paragraphs 85-86, further comprising a plurality of elongate channels that are spaced from one another on said exterior of said container perforator, that are disposed between each adjacent pair of elongate liquid include channels, and that are concave.
88. The spike assembly of paragraph 87, wherein a width of each elongate channel of said plurality of elongate channels progressively decreases proceeding proximally away from an end closest to said distal tip of said container perforator.
89. The spike assembly of any of paragraphs 79-84, further comprising a plurality of elongate channels that are spaced from one another on said exterior of said container perforator and that are concave.
90. The spike assembly of paragraph 89, wherein a width of each elongate channel of said plurality of elongate channels progressively decreases proceeding away from an end closest to said distal tip of said container perforator.
91. The spike assembly of any of paragraphs 79-83, wherein said elongate spike comprises a perforator extension and a container perforator installed on a distal end of said perforator extension, wherein said perforator extension extends from said container perforator to said proximal end, and wherein said perforator extension and said container perforator each comprise said first liquid passage and said first air passage.
92. The spike assembly of paragraph 91, wherein said container perforator comprises at least 3 retention sections that are spaced from one another, that extend away from said central reference axis proceeding in a direction of said proximal end, and that have a free end that is spaced radially outward from said perforator extension.
93. The spike assembly of paragraph 92, wherein a proximal section of each said retention member curves away from said central reference axis proceeding to a corresponding said lower free end.
94. The spike assembly of any of paragraphs 92-93, wherein said spike assembly further comprises a base that in turn comprises a base distal surface, wherein said perforator extension extends from said base distal surface.
95. The spike assembly of paragraph 94, wherein an outer diameter of said base is greater than an outer diameter of each of said perforator extension and said container perforator.
96. The spike assembly of any of paragraphs 94-95, wherein said free end of each said retention member is disposed in spaced relation to said base distal surface.
97. The spike assembly of any of paragraphs 91-96, wherein a separation force is less than a removal force, said separation force is a minimum force required to separate said container perforator from said perforator extension, said removal force is a minimum force required to remove said container perforator from the medical fluid container when said spike assembly is in an installed configuration, and said separation force is less than said removal force.
98. A spike assembly for fluidly accessing a medical fluid container, said spike assembly comprising an elongate spike that in turn comprises:
   a central reference axis that coincides with a length dimension of said elongate spike;
   a distal end and a proximal end that are spaced from one another along a length dimension of said elongate spike;
   perforator extension;
   a container perforator installed on a distal end of said perforator extension, wherein said perforator extension proceeds proximally from said container perforator to said proximal end;
   a first liquid passage that intersects an exterior of said spike and that proceeds along said length dimension on an interior of said elongate spike;
   a first air passage that intersects said exterior of said elongate spike and that proceeds along said length dimension on said interior of said elongate spike;
   wherein said perforator extension and said container perforator each comprise said first liquid passage and said first air passage; and
   wherein said container perforator comprises at least 3 retention sections that are spaced from one another, that extend away from said central reference axis proceeding in a direction of said proximal end, and that have a free end that is spaced radially outward from said perforator extension.
99. The spike assembly of paragraph 98, wherein a proximal section of each said retention member curves away from said central reference axis proceeding to a corresponding said free end.
100. The spike assembly of any of paragraphs 98-99, wherein said spike assembly further comprises a base that in turn comprises a base distal surface, wherein said perforator extension extends distally from said base distal surface.
101. The spike assembly of paragraph 100, wherein an outer diameter of said base is greater than an outer diameter of each of said perforator extension and said container perforator.
102. The spike assembly of any of paragraphs 100-101, wherein said free end of each said retention member is disposed in spaced relation to said base distal surface.

### BRIEF DESCRIPTION OF THE DRAWINGS

Figure 1 is a perspective view of a medical container assembly that uses a medical container adapter assembly to provide a fluid connection between a medical fluid container and medical tubing.
Figure 2 is a perspective view of the medical container adapter assembly of Figure 1, and that utilizes a single-use spike assembly and a multi-use spike interface cover that are detachably connected.
Figure 3 is a side view of the spike interface cover of Figure 2.
Figure 4 is a top view of the spike interface cover of Figure 2.
Figure 5 is an enlarged, perspective view of the spike interface cover of Figure 2.
Figure 6 is a cross-sectional view of the medical container adapter assembly of Figure 2 installed on a medical fluid container.
Figure 7 is a perspective view of the medical container adapter assembly of Figure 2 with a perforator cover/blister pack installed on the spike assembly.
Figure 8 is a perspective view of the perforator cover/blister pack of Figure 7.
Figure 9 is one cross-sectional view of the spike assembly of Figure 2.
Figure 10 is a perspective view of a lower or proximal end of the spike assembly of Figure 2.
Figure 11 is perspective view of a side of a variation of the spike assembly of Figure 2.
Figure 12 is a perspective view of a container perforator used by the spike assembly of Figure 11.
Figure 13A is a perspective view of a container perforator used by the spike assembly of Figure 2.
Figure 13B is an enlarged perspective view of a distal end of the container perforator of Figure 13A.
Figure 13C is a perspective view of a proximal end of a variation of the container perforator of Figure 13A.
Figure 13D is a perspective view of the container perforator of Figure 13C.
Figure 13E is a cross-sectional view of the container perforator of Figure 13A.
Figure 14 is a side view of the spike assembly of Figure 2.
Figure 15A is a cross-sectional view of the spike assembly of Figure 2.
Figure 15B is another cross-sectional view of the spike assembly of Figure 2.
Figure 15C is a cross-sectional view taken along line A-A in Figure 15B.
Figure 16 is a cross-sectional view of a base of the spike assembly of Figure 2, taken along line B-B in Figure 6.
Figure 17 is a perspective view of an interior side of an end cap for the spike assembly of Figure 2.
Figure 18 is a perspective view of an exterior side of the end cap of Figure 17.
Figure 19A is a perspective view of an exterior side of a variation of the end cap of Figure 17, which uses a plurality of segments that separate along a predetermined path.
Figure 19B is a perspective view of an interface or insert used by the cover of Figure 19A.
Figure 19C is a top view of the interface of Figure 19B.
Figure 19D is a cross-sectional view of the interface of Figure 19C, taken along line D-D.
Figure 20 is a perspective view of the medical container assembly of Figure 1, with the spike interface cover having been detached from the spike assembly.
Figure 21 is a cross-sectional view of the medical container assembly of Figure 20.

### DETAILED DESCRIPTION

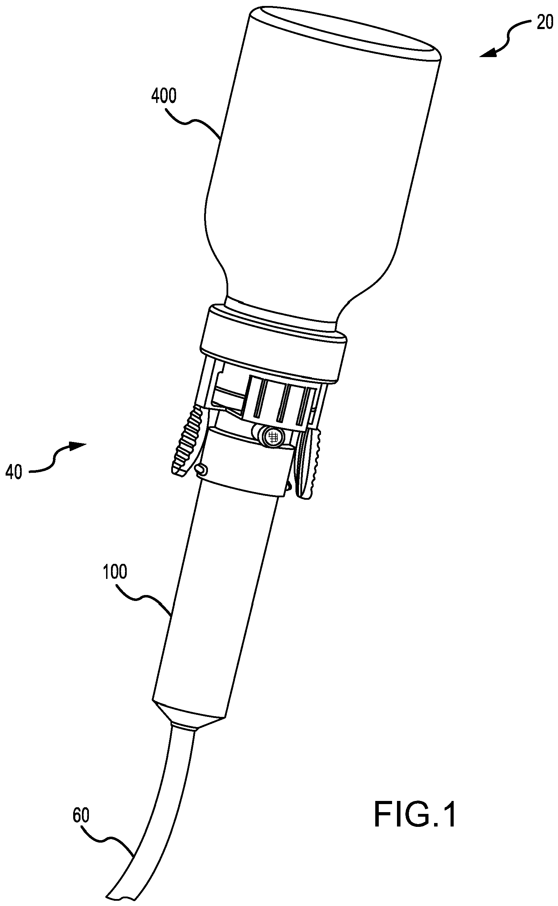

A medical container assembly is shown in Figure 1 and is identified by reference numeral 20. The medical container assembly 20 includes a medical fluid container 400 and a medical fluid container adapter set or assembly 40 (which hereafter may also be referred to as a "medical container adapter assembly 40"). The medical fluid container 400 may contain any appropriate medical fluid, such as contrast media, and is sized to accommodate use for multiple injections (e.g., multiple injections of one, but more typically multiple patients). That is, the medical fluid container 400 has a sufficient storage volume such that the medical fluid container 400 need not be changed from injection to injection. The medical fluid container 400 may be of any appropriate configuration, may be formed from any appropriate material or combination of materials, and may be of any appropriate volume. Examples for the medical container 400 include bottles (e.g., glass; rigid), bags (e.g., flexible), and the like.

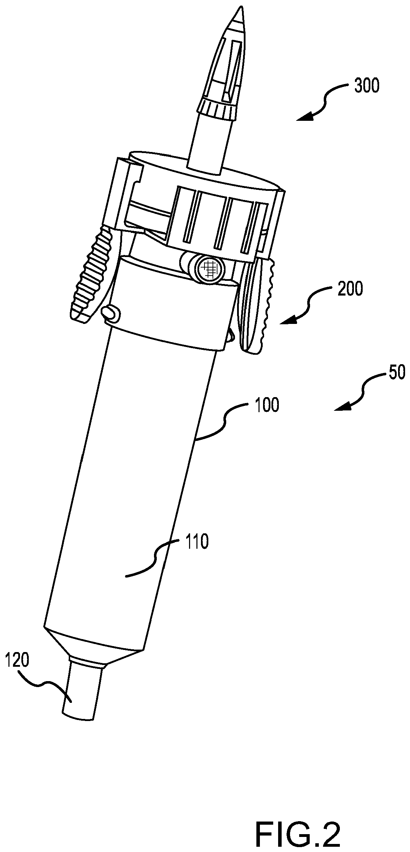

An example of a medical container adapter assembly 40 is shown in Figures 1 and 2. The medical fluid container adapter assembly 40 comprises a first connector part and a second connector part. In the following description, the first connector part is a spike assembly and the second connector part is a spike interface cover. Therefore, the medical fluid container adapter assembly 40 includes a spike assembly 300 (e.g., the first connector part), a spike interface cover 200 (e.g., the second connector part) for the spike assembly 300, and a container (e.g., a drip chamber) 100. The spike assembly 300 is configured to fluidly connect with the medical container 400, and the spike interface cover 200 is configured to detachably connect with the spike assembly 300. A multi-use assembly 50 may include the spike interface cover 200 and container 100. Medical tubing or conduit 60 (e.g., flexible/pliable) is interconnected with and extends from container 100 (e.g., to an injection device (e.g., a power injector), such as to a syringe used by the injection device). Generally, the spike assembly 300 is a single use connector part configured for single-use, that is for use with a single medical container 400. Stated another way, the spike assembly 300 is configured so as to reduce the potential that the spike assembly 300 can be installed on one medical fluid container 400, used for multiple injections (of one or more patients), to be removed from the container 400 (e.g., in an empty or reduced liquid volume condition), installed on a separate medical fluid container 400 (e.g., in a "full" condition), and then used for multiple injections (of one or more patients). The configuration of the spike assembly 300 should reduce the potential for contamination.

Continuing to refer to Figures 1 and 2, the container 100 of the adapter assembly 40 has two distinct areas - a tube bonding area (or a medical conduit/tubing interface) 120 for assembly on the medical tubing 60 (e.g., to accommodate connection of the medical tubing 60 with the container 100) and a chamber part 110 for receipt of medical fluid prior to injection (e.g., useful for priming the medical tubing 60 and visualising the liquid flow). The spike interface cover 200 may be secured to the upper part of the chamber 110 in any appropriate manner, such as by being bonded (e.g., by a solvent or any appropriate adhesive), heating sealing, RF sealing, or the like. The medical tubing interface 120 may be incorporated in any appropriate manner so as to accept a flow of liquid from the spike interface cover 200.

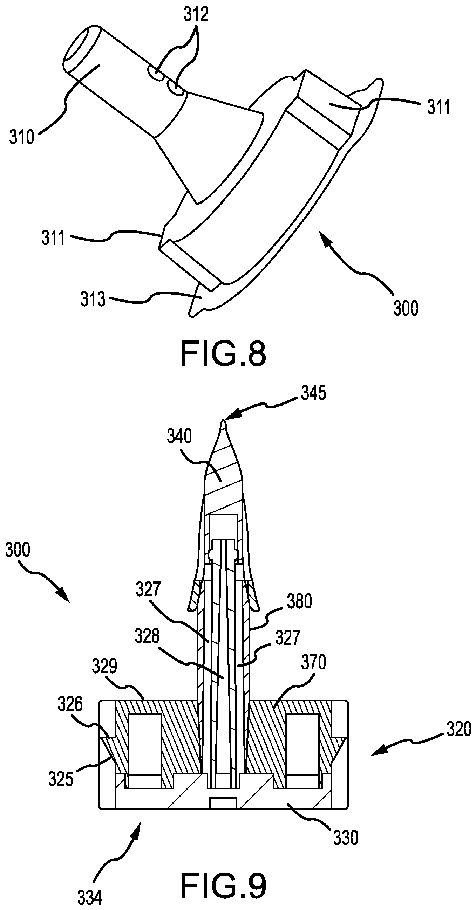

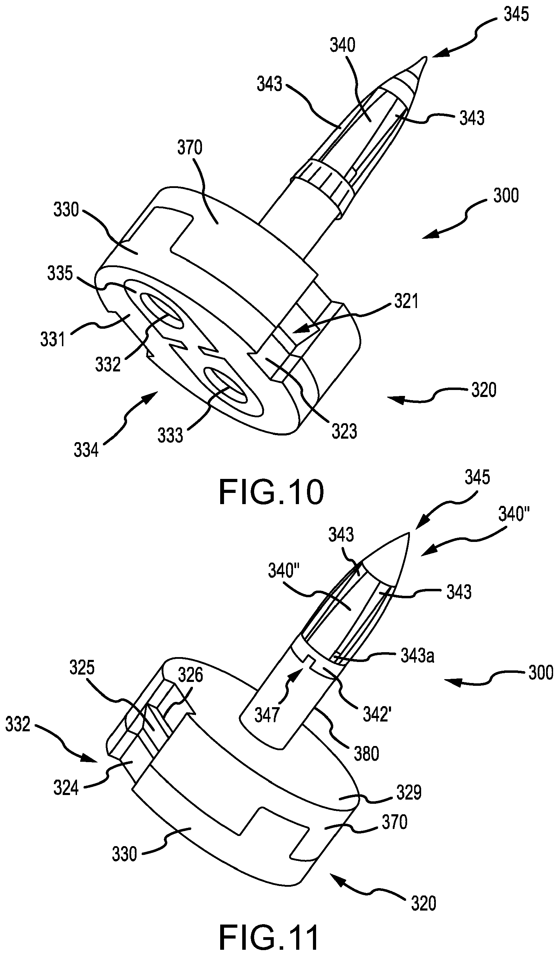

As will be discussed in more detail below, the spike assembly 300 and comprises (e.g., in relation to Figure 9 and 10) a container perforator 340, a liquid passage 327 and an air passage 328. The spike assembly 300 also includes an interface 335 on a proximal end 324 thereof and having a port 332 (airflow) and a port 333 (liquid) that are each fluidly connectable with the container perforator 340 of the spike assembly 300 that is disposed/extends within the interior of the medical fluid container 400. The interface 335 is how/where the spike interface cover 200 fluidly connects with the spike assembly 300.

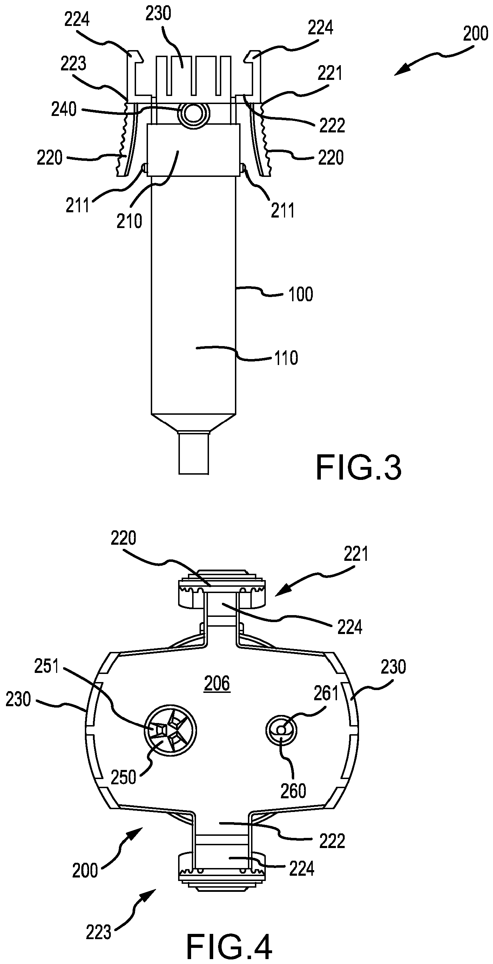

The spike interface cover 200 is illustrated in Figures 1-5. The spike interface cover 200 is an interface that is usable for an extended period of time of any appropriate duration (e.g., for 12 or 24 hours), to connect the spike interface cover 200 to a single-bottle spike assembly 300 installed on a medical fluid container 400. The spike interface cover 200, however, may be removed from one spike assembly 300 (installed on one medical fluid container 400), and may be installed on another spike assembly 300 (installed on an entirely separate medical fluid container 400). Again, each medical fluid container 400 may be used for multiple injections (e.g., of multiple patients). This may be repeated any appropriate number of times (e.g., the spike interface cover 200 may be used with any appropriate number of medical fluid containers 400, each having a dedicated spike assembly 300). As such, the spike interface cover 200 may be referred to as reusable, multi-use, or the like.

The spike interface cover 200 may be characterized as including a body 210 of any appropriate configuration (e.g., an integrally-formed structure; formed by one or more housings/sections that are appropriately connected to one another). The spike interface cover 200 is detachably connectable with the spike assembly 300. That is, the spike interface cover 200 may be characterized as being changeable or movable throughout a range extending from a connecting, latching, or closed configuration (where the spike interface cover 200 is mounted to the spike assembly 300, and which may restrain relative motion between the spike interface cover 200 and spike assembly 300 in at least one dimension - e.g., along a length dimension of the spike assembly 300), to an open or unlatched configuration (to accommodate removal of the spike interface cover 200 from the spike assembly 300, such as by relative movement of these components away from one another along an axial path) - all without damaging the structural integrity of the spike interface cover 200. In this regard, the spike interface cover 200 includes multiple clips or clamps that are movably integrated with the body 210 of the spike interface cover 200 and that detachably engage the spike assembly 300 (in the connecting or latching configuration). Any appropriate number of such clips may be used by the spike interface cover 200. In the illustrated embodiment the spike interface cover 200 uses a pair of clips 221, 223 and these clips 221, 223 are disposed in opposing relation to one another (e.g., disposed or spaced 180° apart about a central reference axis of the adapter assembly 40 and that coincides with a length dimension of the adapter assembly 40).

The clips 221, 223 each include a finger rest area or actuating surface/section 220 and a head 224 that are spaced from one another. The heads 224 of the clips 221, 223 may be of different widths to provide a positional registration feature relative to the spike assembly 300 (e.g., such that the spike interface cover 200 can only be installed in one orientation on the spike assembly 300), and as will be discussed in more detail below. A hinge 222 of any appropriate type interconnects the clips 221, 223 with the body 210 of the spike interface cover 200 and is disposed between the actuating surface 220 and the corresponding head 224 of each clip 221, 223. The hinge 222 may be in the form of a living hinge (e.g., the clips 221, 223 may be integrally formed with the body 210 of the spike interface cover 200, and where the hinge 222 is an elastically-deformable section that allows each clip 221, 223 to move (e.g., pivot) about its corresponding hinge 222. Each clip 221, 223 may be biased to a position where the clips 221, 223 will detachably engage the spike assembly 300. The range of movement may be characterized as being from a neutral position (e.g., where the clips 221, 223 are in a non-elastically deformed state) to an open position where the heads 324 of the clips 221, 223 are disengaged from the spike assembly 300 so as to allow the spike interface cover 200 to be removed from the spike assembly 300 (via relative movement between the spike interface cover 200 and spike assembly 300 away from each other and along an axial path). The noted neutral position may correspond with a clipping, clamping, latching, or locking position (relative to the spike assembly 300, and where the heads 324 of the spike interface cover 200 are latched or locked to the spike assembly 300, although the noted latching or locking position could be somewhere between the neutral position and open position.

The spike interface cover 200 may include a stop 211 for each clip 221, 223. The stops 211 are in the form of a protrusion on an outer perimeter of the body 210 of the spike interface cover 200. The role of the stops 211 is to limit movement of the clips 221, 223 relative to the spike body 210 to within an elastic deformation range for the hinges 222 (e.g., when in the form of a living hinge). In any case, the pinch pressure between a thumb and finger of a user allows simultaneous opening of the clips 221 and 223 to release the spike interface cover 200 from the spike assembly 300.

An upper or distal end 202 of the spike interface cover 200 includes a receptacle 204 (e.g., a concave space) in which at least part of the spike assembly 300 may be disposed in the assembled configuration (e.g., a proximal end section or portion). In this regard, the spike interface cover 200 includes a recessed base distal surface 206 which may be a flat or planar surface. A part of each clip 221, 223 extends upwardly or distally beyond the base distal surface 206. A plurality of upper wall segments 230 also extend upwardly or distally from the base distal surface 206, protruding distally from the base distal surface 206. The plurality of upper wall segments 230 are spaced from one another. One or more upper wall segments 230 are disposed between the clips 221, 223 on one side of the spike interface cover 200, and one or more upper wall segments 230 are disposed between the clips 221, 223 on an opposite side of the spike interface cover 200. Stated another way, one or more upper wall segments 230 are disposed between the clips 221, 223 proceeding in one direction about an outer perimeter of the spike interface cover 200, and one or more of upper wall segments 230 are disposed between the clips 221, 223 proceeding in an opposite direction about the outer perimeter of the spike interface cover 200. Having multiple upper wall segments 230 be disposed in spaced relation to one another and on each side of the spike interface cover 200 provides the advantage or benefit of increasing the ability to maintain/retain the overall shape (e.g., outer perimeter profile) of the receptacle 204 after completion of fabrication (compared to having a pair of continuous walls disposed in opposing relation to one another and in place of the two sets of upper wall segments 230 (again where each such set includes multiple upper wall segments 230 that are spaced from one another)), including for the case of an injection-molded spike interface cover 200. This in turn increases the ability for the spike interface cover 200 to receive the spike assembly 300 in a desired manner. Having two different sets of multiple upper wall segments 230 that are spaced from one another within each set, and with each set being disposed in opposing relation to the other set, may be characterized as reducing the potential for ovalization of the shape of an outer perimeter of the receptacle 204 during/after injection molding. The height of the upper wall segments 230, along with the protruding portion of the clips 221, 223, reduces the risk of contact with perforators 250, 260 (discussed below) of the spike interface cover 200. This contact can be physical with a table or manual with a finger, for example. That is, the perforators 250, 260 of the spike interface cover 200 are recessed relative to the distal or free ends of the upper wall segments 230/the heads 224 of the clips 221, 223, which should reduce the potential for the perforators 250, 260 becoming contaminated between installation of the spike interface cover 200 on different spike assemblies 300, or stated another way, a distal end of each of the perforator 250 for the liquid passage and the perforator 260 for the air passage are disposed closer to the base distal surface 206 than a distal end of each of said plurality of upper wall segments 230. The perforator 250 for the liquid passage and the perforator 260 for the air passage are located inwardly of an outer perimeter collectively defined by said plurality of upper wall segments 230.

The spike interface cover 200 comprises a liquid passage 214 and an air passage 261. The liquid passage 327 of the spike assembly 300 and the air passage 328 of said spike assembly 300 are fluidly connected with said liquid passage 214 and said air passage 261, respectively, of said spike interface cover 200. An active area 260 of the spike interface cover 200 accommodates the transfer of air from the outside of the medical fluid container 400 to the inside of the medical fluid container 400, by opening/perforating an area provided for this purpose on the spike assembly 300. For example, he active area 260 of the spike interface cover 200 may be in the form of a perforator 260 that protrudes or extends distally from the base distal surface 206. The perforator 260 for the air passage is fluidly connected to said air passage 261. The perforator 260 for the air passage includes an air passage that intersects with an exterior of the perforator 260, forming a part of the air passage 261 of the spike interface cover 200. The distal end of the perforator 260 is again recessed relative to the distal ends of the upper wall segments 230 and clips 221, 223. Stated another way, the distal end of the perforator 260 is located between the base distal surface 206 and the distal ends of the upper wall segments 230 and clips 221, 223 along the length dimension of the spike interface cover 200 (the perforator 260 extending distally from the base distal surface 206 in this length dimension).

A vacuum is created when withdrawing liquid from the medical fluid container 400, and it is necessary to draw in outside air (air that is external to the medical fluid container 400) to the interior of the medical fluid container 400 to allow for dispensing of liquid from the medical fluid container 400 at a desired rate and/or for a desired amount of time. The air passage 261 extends from a location on the exterior of the perforator 260 to another location (e.g., proximal of the perforator 260) on an exterior of the body 210 of the spike interface cover 200. As external or outside air flowing through the air channel 216 is arriving from an uncontrolled environment (ambient air), it should be filtered. In this regard, the spike interface cover 200 includes a filter 240 with an appropriate filtering media 242. This filter 240 may be overmolded relative to the body 210 of the spike interface cover 200, may be disposed in a filter port of the spike interface cover 200, or both. This filter 240 may be force-fitted into an aperture of the body 210 of the spike interface cover 200 that is in direct communication with the air passage 261. The diameter of the air passage 261 should be sufficient, for instance on the order of 1 mm or 1.5 mm, to allow for sufficient air flow so as to limit the vacuum phenomenon in the medical fluid container 400 as liquid is being dispensed therefrom (through the spike assembly 300, through the spike interface cover 200, through the container 100, and into the tubing 60.

An active area 250 of the spike interface cover 200 is the part that accommodates the withdrawal of liquid from the inside of the medical fluid container 40 (e.g., for transfer to an injector syringe), by opening/perforating an area provided for this purpose in the spike assembly 300. The active area 250 of the spike interface cover 200 may be in the form of a perforator 250 that protrudes distally from the base distal surface 206 and that includes a liquid passage 214. The perforating power of the perforator 250 may be enhanced by having a distal end or tip 252 being in the form of a small (e.g., convex) radius 252. This radius may be on the order of 0.05 mm. The distal tip or end of the perforator 260 may be similarly defined. As in the case of the perforator 260, the distal end or tip 252 of the perforator 250 is recessed relative to the distal ends of the upper wall segments 230 and clips 221, 223. Stated another way, the distal tip 252 of the perforator 250 is located between the base distal surface 206 and the distal ends of the upper wall segments 230 and clips 221, 223 along the length dimension of the spike interface cover 200 (the perforator 250 extending distally from the base distal surface 206 in this length dimension).

The perforator 250 is provided with at least one passage 251 that intersects with an exterior of the perforator 250, and that is in fluid communication with a liquid passage 214 (the passage(s) 251 prolongs the liquid passage 214 and may be characterized as being part of a liquid passage for the spike interface cover 200). Using multiple passages 251 accommodates reducing the size (e.g., diameter) of the passages 251 within the perforator 250 while still accommodating a sufficient flow rate of liquid from the medical container 400 and without adversely affecting the structural integrity of the spike interface cover 200 (more specifically the perforator 250) to an undesired degree. Three passages 251 could be utilized and evenly distributed, i.e. every 120°. The cross-sectional area, through which the fluid flows out of the medical fluid container 400, is critical in the pressure drop that will be generated by the passage of fluid. This is even more important in the transfer of medium-viscosity products such as a contrast agent. It is preferable to obtain the best compromise between the fluid flow section and the external diameter of the perforator 250. The larger the diameter of the perforator 250, the more complex it will be to integrate it into the system - bearing in mind that it will also be desirable to have sufficient peripheral sealing between the perforator 250 and the interface 335 of the spike assembly 300 (e.g., Figure 10, discussed below).

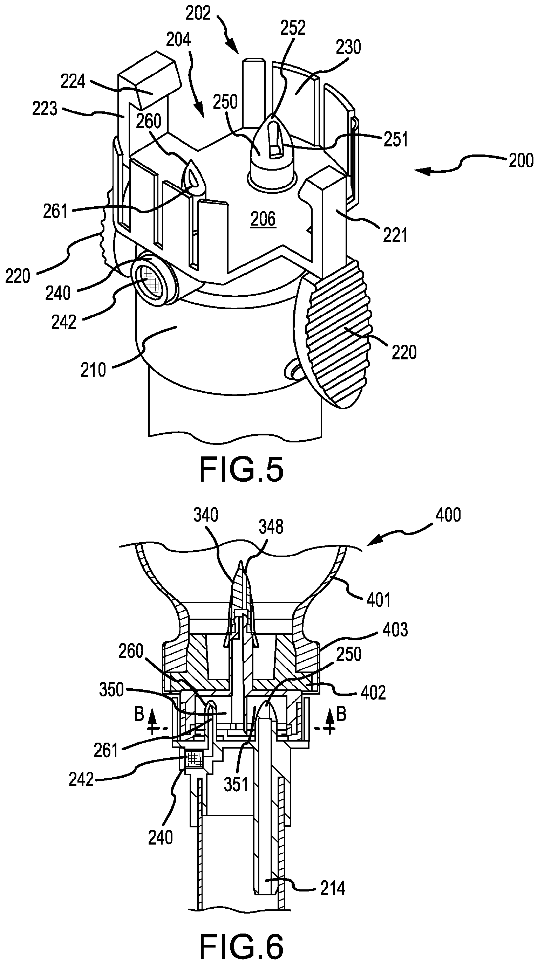

Figure 6 shows the spike interface cover 200 being attached to the spike assembly 300, with the spike assembly 300 in turn being installed on the medical fluid container 400 (where a portion of the spike assembly 300 extends into the interior liquid-containing volume of the medical fluid container 400, namely a container perforator 340). The medical fluid container 400 is mainly composed of a body 401 (e.g., glass) and a cap, closure, seal, stopper, or septum 402 that is disposed in an outlet (e.g., a neck) of the container 400. The septum 402 may be formed from a flexible, deformable and perforable material, and may be press-fit within the outlet from the container 400. The septum 402 may be held relative to the body 401 of the medical fluid container 400 by an aluminium ring 403. This ring 403 may be partially removed when using the medical fluid container 400 and before connecting the tubing 60.

The spike assembly 300, spike interface cover 200, container 100, and medical tubing 60 may be preassembled and disposed within common packaging that is appropriate for the application (e.g., to maintain sterility). However, the spike assembly 300 could be separately packaged, and the spike interface cover 200, container 100, and medical tubing 60 could collectively be separately packaged. Again, the spike assembly 300 directly interfaces with the medical fluid container 400. Details of the spike assembly 300 will now be discussed in relation to Figures 2 and 7-11.

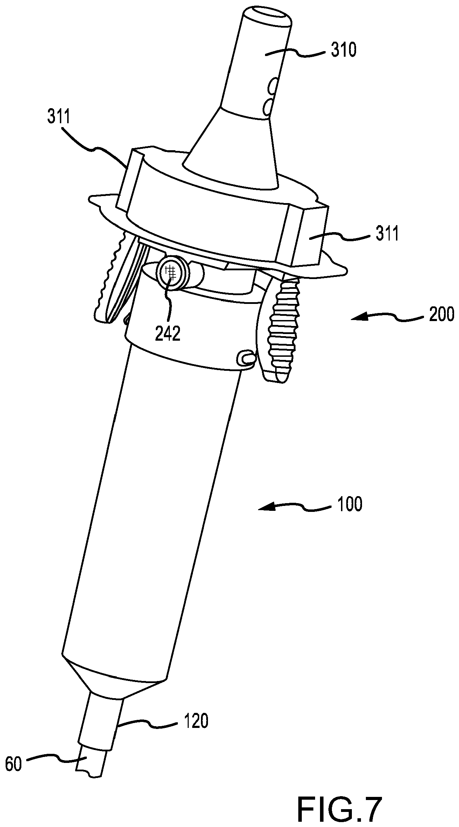

The spike assembly 300 assembly may be delivered in a blister pack 310 (e.g., packaging) and as shown in Figures 7 and 8. A proximal end of the blister pack 310 may be closed by a porous-paper or Tyvek^{®}-type non-woven cover. This porous side may be secured to the blister pack 310 at a flange 313. That is, the removable closure of the blister pack 310 coincides with a side of the spike assembly 300 on which the spike interface cover 200 is installed (the "underside" of the flange 313 shown in Figure 8).

After opening the proximal end of the blister pack 310 by removing/peeling the porous side (to expose a lower or proximal end of the spike assembly 300 disposed within the blister pack 310), the spike interface cover 200 and spike assembly 300 may be detachably interconnected. The blister pack 310 has a pair of oppositely disposed receivers 311 to receive the oppositely disposed clips 221 and 223 of the spike interface cover 200 so as to clip or latch the spike interface cover 200 onto the spike assembly 300. The blister pack 310, the spike assembly 300, the spike interface cover 200, the container 100, and tubing 60 can thus remain on standby (and with the medical tubing 60 being connected to a syringe of the injector) while waiting for the spiking of a new medical fluid container 400.

A container perforator 340 and at least part of the spike body 320 of the spike assembly 300 (e.g., Figure 9) remain protected from contact (and from the surrounding environment) by the blister pack 310. Liquid from the medical fluid container 400 flows into the installed spike assembly 300 through the container perforator 340 and as will be discussed in more detail below. As such, the spike assembly 300 and spike interface cover 200 may be assembled in a more sterile manner. The lower end of the spike assembly 300 need only be exposed for a very short time after the removal of the cover of the blister pack 310 and the installation of the spike interface cover 200 onto the spike assembly 300. The blister pack 310 may be held in place on the spike assembly 300 by one or more small depressions or detents 312 that interfere with the two components (e.g., a pair of depressions 312 could provide an interference fit between the blister pack 310 and the spike assembly 300 on opposite sides of its perforator 340). These small depressions 312 can be made after placing the spike assembly 300 in the blister pack 310 or beforehand during the manufacture of the blister pack 310. In one embodiment, the blister pack 310 may be thermoformed. The blister pack 310 could also be made by plastic injection (e.g., injection molding). The blister pack 310 can be removed from the spike assembly 300 immediately prior to installing the same on the medical fluid container 400.

The spike assembly 300 is shown in Figures 9 and 10 and includes three main parts: the above-noted container perforator 340, the above-noted spike body 320, and a cover or end cap 330. The spike body 320 may include a base 370 having an upper or distal base surface 329 (e.g., flat or planar), along with a perforator extension 380 that extends distally from this base distal surface 329. The container perforator 340 protrudes distally relative to said first base distal surface 329. The container perforator 340 is disposed on/mounted to a distal end of the perforator extension 380. The container perforator 340 is thereby spaced from the base distal surface 329 by a proximal section of the perforator extension 380 (e.g., which provides a sealing location between the spike assembly 300 and the septum 402 of the medical container 400 - discussed in more detail below). The container perforator 340 and the perforator extension 380 could also collectively be referred to as a "perforator." The perforator extension 380 comprises at least a part of the liquid passage 327 and the air passage 328. The container perforator 340 is interconnected with the perforator extension 380.

The spike body 320 and end cap 330 may be collectively referred to as a "first body" for the spike assembly 300. Such a "first body" could also include the perforator extension 380. In any case, the spike body 320 and the end cap 330 may be tightly connected, such as by bonding, ultrasonic welding, or the like. A clipping or snap-lock arrangement between these two parts 320 and 330 would also be possible, but may require the use of a gasket. Any appropriate way of attaching the spike body 320 to the end cap 330 may be utilized. The container perforator 340 and the spike body 320 could be bonded or clipped together (e.g., via a snap-lock arrangement). The perforator 340 could also be integrated into the spike body 320 (e.g., such that the container perforator 340 and spike body 320 are of an integral construction; to make a single part cast in one piece).

The spike assembly 300 is targeted for use with a single medical fluid container 400. The spike assembly 300 is not intended to be installed on one medical fluid container 400, and to thereafter be removed from this medical fluid container 400 and installed on another medical fluid container 400, which could increase the potential for contamination/cross-contamination. The configuration of the spike assembly 300 reduces the potential for use of the spike assembly 300 on sequential medical fluid containers 400. Generally, the spike assembly 300 is configured such that the amount of force required to detach/remove the entire spike assembly 300 from a medical fluid container 400 is greater than the amount of force required to detach the container perforator 340 from the spike body 320. The spike assembly 300 is configured such that said container perforator 340 will separate from the spike body 320 before said spike body 320 will separate from the medical fluid container 400 when the medical fluid container adapter assembly 40 is in an installed configuration. In order to further reduce the potential of incorrect use of the spike assembly 300 and the withdrawal of the spike assembly 300 from the medical fluid container 400 before spiking a new medical fluid container 400, the container perforator 340 is equipped with a proximal skirt whose function is to at least reduce the potential of withdrawal of the container perforator 340 from the medical fluid container 400 (e.g., incorporating a "harpoon" functionality in the structure of the container perforator 340). This harpoon function will be detailed later.

The separation of the spike body 320 and the container perforator 340 could then be done by a breakage area whose value would be predefined. Thus, by defining:
- F0: a force to withdraw a standard spike from a medical container 400,
- F1: a force to withdraw a container perforator 340 equipped with a harpoon function from a medical container 400,
- F2: a force of uncoupling of the container perforator 340 and the spike body 320, F0 < F2 < F1

An attempt to withdraw the spike assembly 300 from a medical fluid container 400, by exertion of a significant force on the spike assembly 300, should result in an uncoupling of the container perforator 340 and spike body 320 before withdrawal of the container perforator 340 from the septum 402 of the medical fluid container 400. The container perforator 340 should thus remain in the interior of the medical fluid container 400. The spike assembly 300 without its container perforator 340 (after separation of a portion thereof from the container perforator 340) can no longer be used to spike a new medical fluid container 400. The shape of the spike body 320 alone should not allow perforation of a septum 402 of a medical fluid container 400. Summarily, the spike assembly 300 is configured such that the container perforator 340 should separate from a remainder of the spike assembly 300 before the container perforator 340 could be withdrawn from the septum 402 of the medical fluid container 400 by attempting to pull the spike assembly 300 away from the medical fluid container 400.

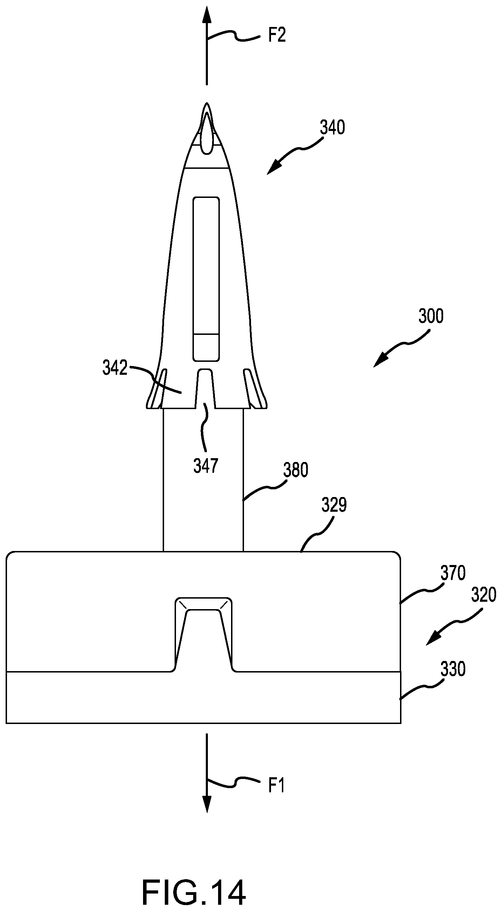

Figure 14 (discussed in more detail below) shows the above-noted forces F1 and F2 above. If a force is exerted on the spike assembly 300 in the direction of the arrow for force F1 shown in Figure 14, the interface between the container perforator 340 and the septum 402 of the medical fluid container 400 will exert a force on the container perforator 340 that is in the direction of the arrow for force F2 shown in Figure 14. Once the magnitude of the force being exerted on the container perforator 340 by the interface with the septum 402 (where this force is in the direction of the arrow for force F2 shown in Figure 14) reaches the above-noted magnitude of force F2 (and as the force continues to be exerted on the spike assembly 300 in the direction of the arrow for force F1), the container perforator 340 should separate from the remainder of the spike assembly 300. At the time of this separation of the container perforator 340 from the spike body 320, the force that continues to be exerted on the spike assembly 300 in the direction of the arrow for force F1 would not yet have reached the above-noted magnitude of force F1. As such, the container perforator 340 should separate from the spike body 320 before the container perforator 340 could be entirely withdrawn from the septum 402 (and with the container perforator 340 remaining intact with the spike body 320).

The container perforator 340 of the spike assembly 300 therefore performs two distinct functions - perforation of a septum 402 of a medical container 400, and protection against withdrawal and therefore reuse of the spike assembly 300. Two-stage protection is provided by the spike assembly 300 - increasing the standard withdrawal force of the perforator 340 (e.g., via the harpoon function addressed below) and, if the force exerted by the operator reaches a certain threshold, uncoupling the container perforator 340 and the spike body 320.

As noted above, the spike assembly 300 is attached and held on the spike interface cover 200 by the clips 221, 223 of the spike interface cover 200. These clips 221 and 223 of the spike interface cover 200 are housed in guides 323 and 324, or channels, that are disposed on an outer perimeter of the spike body 320 of the spike assembly 300. In the case where the clips 221, 223 are disposed in opposing relation to one another on the spike interface cover 200, the guides 323, 324 will similarly be disposed in opposing relation to one another on the outer perimeter of the spike assembly 300. The guides 323, 324 are in the form of concave surfaces on the outer perimeter of the spike assembly 300, namely on the outer perimeter of the spike body 320 and end cap 330 in the illustrated embodiment. Each guide 323, 324 extends from a lower or proximal end 334 of the spike assembly 300 (on which the noted ports 332, 333 of the interface 335 are disposed) toward (and including to) the base distal surface 329 (from which the container perforator 340 extends).

Each catch 321, 322 is disposed within a guide 323, 324. During the attachment or clipping phase, the clips 221, 223 (spike interface cover 200) slide in the corresponding guide 323, 324 (spike assembly 300) until they reach a corresponding catch 321, 322 (on the outer perimeter of the spike body 320 in the illustrated embodiment). The clips 221, 223 of the body 210 of the spike interface cover 200 detachably engage the catches 321, 322, respectively, to detachably connect the spike assembly 300 and the spike interface cover 200. The catches 321, 322 are spaced from one another. Each catch 321, 322 includes a sloping surface or slope 325. Each slope 325 proceeds away from a central reference axis of the spike assembly 300 (corresponding with a length dimension of the spike assembly 300 - the dimension in which the distal base surface 329 and proximal end 334 are spaced from one another) in proceeding in the direction of the base distal surface 329. Stated another way, the slopes 325 diverge relative to this central reference axis proceeding in the direction of the base distal surface 329. In the illustrated embodiment the slope of the surfaces 325 is of a constant magnitude, although such may not be required for one or more applications.

The slopes 325 function as a cam or a camming surface for the clips 221, 223. Generally, as the spike interface cover 200 is moved relative to the spike assembly 300 (e.g., along an axial path corresponding with the longitudinal or length dimension of the adapter assembly 40), the engagement of the heads 224 of the spike interface cover 200 with the corresponding slope 325 of the spike assembly 300 will move the heads 224 (via movement of the clips 221, 223 about their corresponding hinge 222 in one direction, which again may be provided by elastic deformation of the clips 221, 223 at their corresponding hinge 222) toward their respective open positions (away from the central reference axis of the adapter assembly 40) until the heads 224 reach a corresponding clipping or locking surface 326 of the spike assembly 300, at which time the heads 224 move (via movement of the clips 221, 223 about their corresponding hinge 222 in an opposite direction) back toward the central reference axis of the spike assembly 300 (and may remain engaged with the corresponding surface 326) to clip, latch, or lock the spike interface cover 200 to the spike assembly 300. At this time the base distal surface 206 of the spike interface cover 200 may be engaged with the proximal end 334 of the spike assembly 300. This movement of the clips 221, 223 to their respective latching positions again may be provided by the elasticity of their corresponding hinge 222.

As noted, the medical container adapter assembly 40 may be configured such that the spike interface cover 200 can only be installed on the spike assembly 300 in one orientation - where the perforator 250 (liquid) of the spike interface cover 200 is disposed in a chamber 351 (liquid) of the spike assembly 300 and where the perforator 260 (air) of the spike interface cover 200 is disposed in a chamber 350 (air) of the spike assembly 300. The guides 323, 324 can be configured to only accommodate a single relative orientation between the spike assembly 300 and spike interface cover 200 when detachably connected. This may be achieved by having differing widths for the guides 323 and 324 of the spike assembly 300. In one embodiment, one of the guides is 1. 5 times wider than the other. The same logic is applied to the spike interface cover 200 with clips 221, 223 of different widths. In any case, the positional registration function may be configured such that the head 324 of clip 223 (wider) of the spike interface cover 200 cannot fit into the guide 323 of the spike assembly 300 (but can fit into the guide 324), thereby preventing one of the two possible clipping or latching positions. A positional registration function is preferable since it guides the connection of an air passage 328 of the spike assembly 300 with the air passage 261 of the spike interface cover 200 to the air intake, and it similarly guides a liquid passage(s) 327 of the spike assembly 300 to the liquid passage 214 of the spike interface cover 200 (where this liquid passage 214 opens into the main barrel of the container 100).

Access and communication between the various passages of the spike assembly 300 and spike interface cover 200 is provided by the perforation of an insert or interface 335 disposed on the proximal end 334 of the spike assembly 300 (and incorporated by the end cap 330) that interfaces with the spike interface cover 200. The proximal end 334 of the spike body 320 is spaced from the base distal surface 329 of said spike body 320. This interface 335 may be overmolded to a body 331 of the end cap 330, although the interface 335 may be integrated by the end cap 330 in any appropriate manner. The interface 335 is of a first hardness and the spike body 320 is of a second hardness that is greater than said first hardness. The hardness of the interface 335 may be of a smaller magnitude that a hardness of the body 331 of the end cap 330. Generally, the interface 335 may be formed from a pliable/deformable material for engagement with the perforators 250, 260 of the spike interface cover 200. In one embodiment, the hardness of the interface 335 is between 30 ShA to 90 ShA in one embodiment, is between 50 ShA to 70 ShA in one embodiment, and is about 60 ShA in yet another embodiment.

The interface 335 is configured to deform when the perforator 250 for the liquid passage 214 of the spike interface cover 200 is directed into and through a port 333 of the interface 335. The interface 335 is further configured to deform when the perforator 260 for the air passage 261 of the spike interface cover 200 is directed into and through a port 332 of the interface 335. Based upon the noted positional registration function of the medical container adapter assembly 40 discussed above, the perforator 250 of the spike interface cover 200 will be directed into a port 333 of the interface 335 for the spike assembly 300 and will perforate a portion of the interface 335 to accommodate fluid communication with the liquid passage(s) 327 of the spike assembly 300, while the perforator 260 of the spike interface cover 200 will be directed into the port 332 of the interface 335 for the spike assembly 300 to accommodate fluid communication with the air passages 328, 348 of the spike assembly 300. The "softness property" of the interface 335 is part of the sealing characteristic of the contact between the interface 335 of the spike assembly 300 and the perforators 250, 260 of the spike interface cover 200. Generally, the interface 335 of the spike assembly 300 seals against a perimeter of the perforators 250, 260 of the spike interface cover 200. Sufficient sealing between the interface 335 and perforators 250, 260 is required to force all external air to enter the interior of the medical fluid container 400 (by the vacuum in the medical fluid container 400) by passing through the filter 240 of the spike interface cover 200, and similarly forces the vacuum phase in the container 100 to draw in liquid from the medical fluid container 400 (and to not draw in air from the external environment).

Prior to the perforators 250, 260 of the spike interface cover 200 being directed into the corresponding port 333, 332 of the spike assembly 300, the diameter of the ports 333, 332 may be less than the outer diameter of the corresponding perforator 250, 260. The expansion of the ports 333, 332 when directing the corresponding perforator 250, 260 therein expands the interface 335 (which provides one sealing aspect). The interface 335 may also be configured that the perforators 250, 260 are required to puncture/pass through an aligned portion of the interface 335 prior to entering the corresponding chamber 351, 350 of the spike assembly 300 (and that provides another sealing aspect).

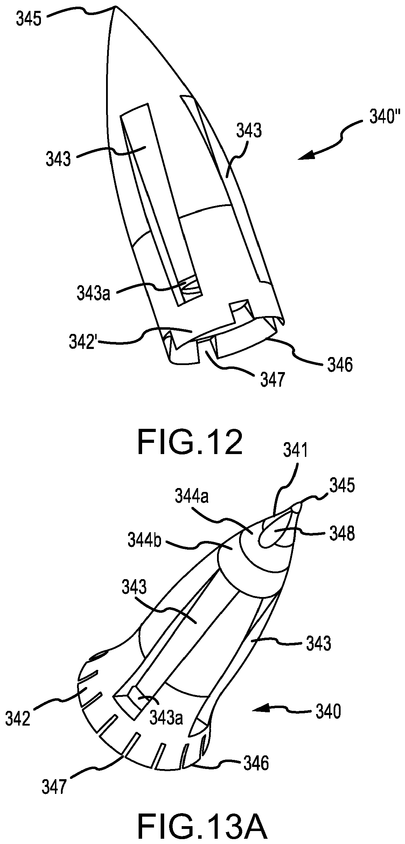

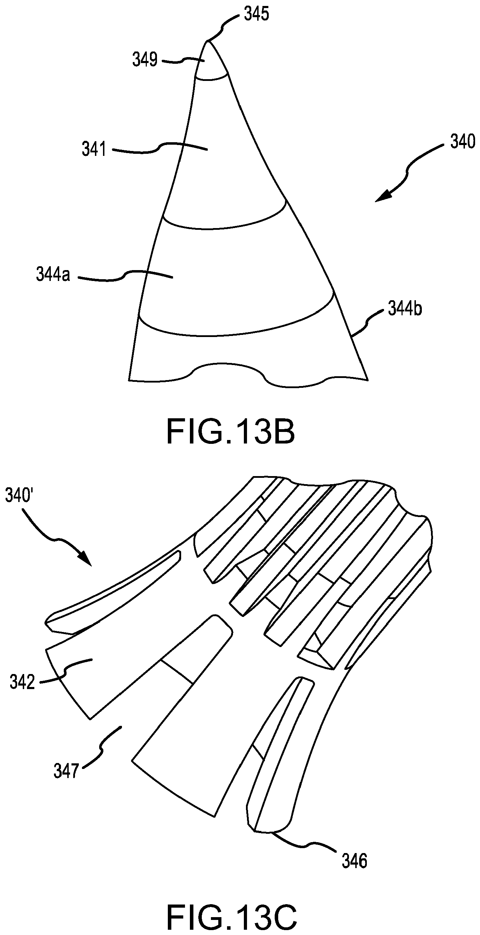

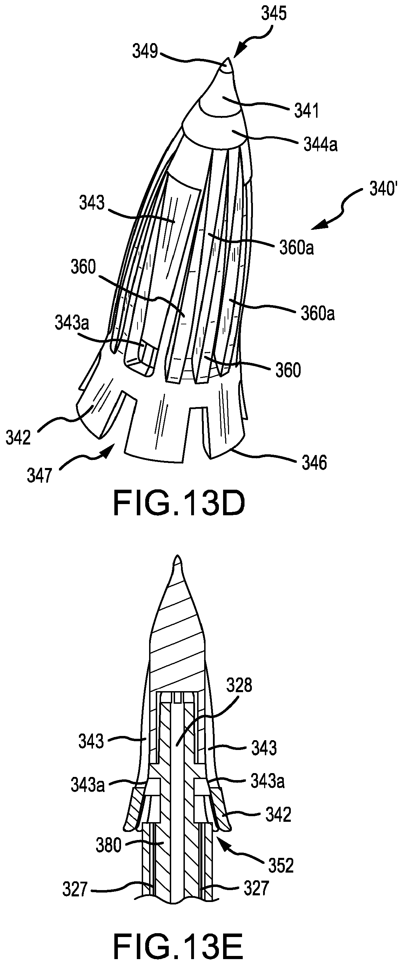

Various details of the container perforator 340 of the spike assembly 300 are also shown in Figures 13A, 13B, 13E and Figure 14. The container perforator 340 may be characterized as including a distal tip 345, a frusto-conical section or a conical trunk 349, a first transition section 341, a second transition section 344a, and a body 344b. The frusto-conical section 349 is located between the distal tip 345 and the first transition section 341 along the length dimension of the container perforator 340 (also corresponding with the spacing between the distal tip 345 and the base distal surface 329 relative to which the container perforator 340 protrudes proximally). The first transition section 341 is located between the frusto-conical section 349 and the multi-use assembly comprising the spike interface cover 200 when the spike interface cover 200 detachably connects with the spike assembly 300. The first transition section 341 is located between the frusto-conical section 349 and the second transition section 344a proceeding along the length dimension of the container perforator 340. The second transition section 344a is located between the first transition section 341 and the body 344b proceeding along the length dimension of the container perforator 340. Generally, the frusto-conical section 349, the first transition section 341, the second transition section 344a, and the portion of the body 344b that extends from the second transition section 344a toward the upper base surface 329 (a distal portion of the body 344) are disposed in different orientations.

The distal tip 345 of the perforator 340 may be defined by a small radius. This radius of the distal tip 345 may be less than 0.15 mm, or may be less than 0.05 mm. For the case of using an injection molding tool to form the spike assembly 300 (or at least the container perforator 340), this area of the injection molding tool (used to define the distal tip 345) may be filled by a moving area, such as a spindle for example. The radius of the distal tip 345 may be advantageously tangent to the section 349, and where this section 349 may be tangent with a radius of that defines where an air passage 348 incorporated by the container perforator 340 intersects an exterior of the container perforator 340 (e.g., Figure 13A), and that will be discussed in more detail below.

The frusto-conical section 349 may be disposed in diverging relation to a central reference axis of the container perforator 340 (centrally disposed and coinciding with the length dimension of the container perforator 340) proceeding in the direction of the first transition section 341 (e.g., a proximal direction). The frusto-conical section 349 may be defined by a constant slope. The perforation function of the perforator 340 is improved by the configuration of the first transition section 341, together with the small radius defining the distal tip 345. This may collectively promote perforation by concentrating the effort on the first tenths of the perforation. In this regard, the first transition section 341 is concave on the exterior of the container perforator 340 and in the length dimension of the container perforator 340 (e.g., the first transition section 341 includes a "curved in" portion proceeding along the length dimension of the container perforator 340). Stated another way, the transition section 341 is a concave surface on the exterior of the container perforator 340 proceeding proximally relative to the frusto-conical section 349.

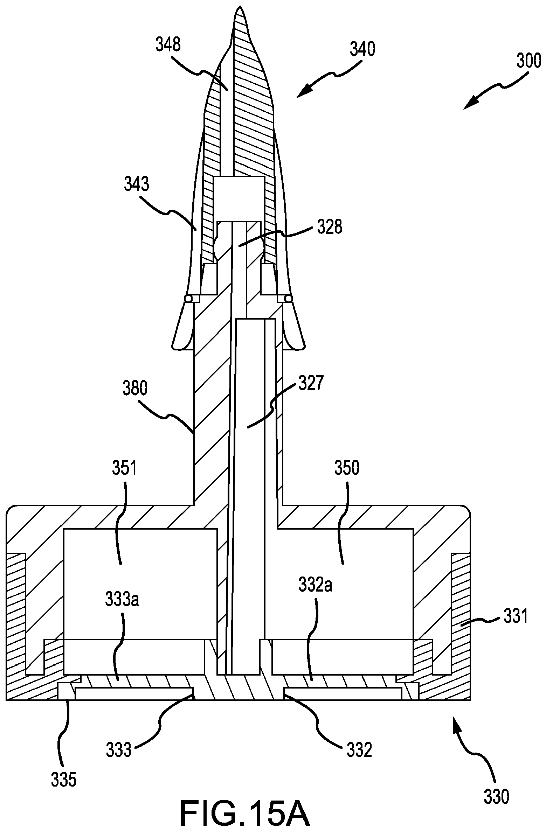

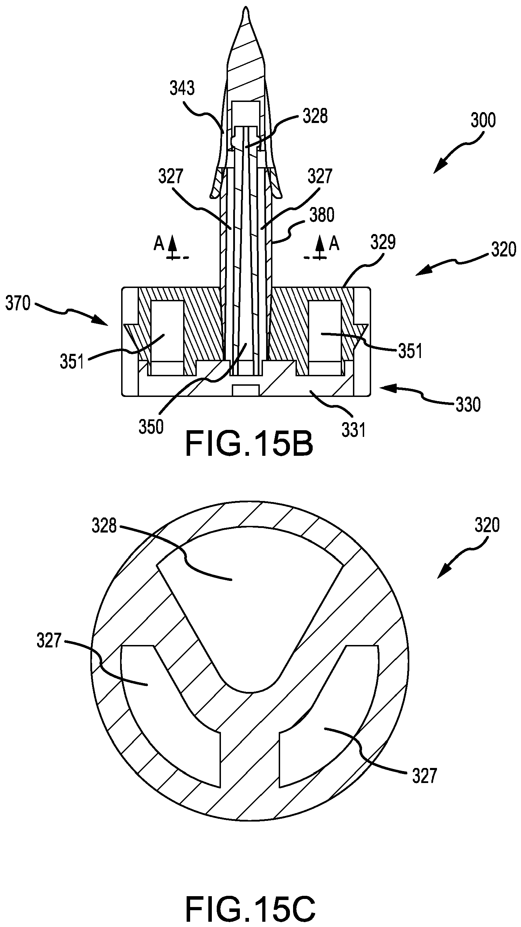

The container perforator 340 further includes a passage 348 that intersects the perimeter of the container perforator 340 in at least the first transition section 341 (e.g. also in the second transition section 344a in one or more embodiments). The passage 348 extends into the container perforator 340 to a passage 328 that intersects with a perimeter of the perforator extension 380 (Figure 13E) on which the container perforator 340 in positioned. The passage 328 extends along/through the perforator extension 380 to an internal chamber 350 in the spike body 320. (e.g., Figures 6 and 15A). A plurality of elongate windows 343 (e.g., concave structures) are circumferentially spaced about an outer perimeter of the container perforator 340. Any appropriate number of windows 343 may be utilized (three in the illustrated embodiment). Each window 343 accommodates flow of liquid from the medical fluid container 400, through a corresponding mouth or port 343a (e.g., proximally disposed in a corresponding window 343), and into one or more passages 327 in the perforator extension 380 (e.g., Figure 15A-15C) that extend along/through the perforator extension 380 and that will be discussed in more detail below. When the medical container assembly 20 is oriented such that the container perforator 340 of the spike assembly 300 is projecting upwardly (e.g., the medical fluid container 400 being positioned above the base 370 of the spike body 320), the intersection of the passage 348 (air outlet to medical fluid container 400) with the exterior of the container perforator 340 should be at least at the same elevation as the mouth or port 343a of each window 343 (liquid inlet to the spike assembly 300), but more preferably the intersection of the passage 348 (air outlet to medical fluid container 400) with the exterior of the container perforator 340 should be at a higher elevation than the mouth or port 343a of each window 343 (liquid inlet to the spike assembly 300).

The choice of a material with a fairly low injection grade may be used for the container perforator 340, and thus contributes to the proper creation of the tapered shape for a distal end section of the container perforator 340. A Methyl methacrylate-Acrylonitrile-Butadiene-Styrene (MABS), Polybutylene Terephthalate (PBT), polycarbonate (PC) or polyolefin type material may be utilized for fabrication of the container perforator 340. A polyolefin or PBT type material may be selected for fabrication of the container perforator 340 due to its low coefficient of friction and therefore a better glide during perforation of the septum 402 of the medical fluid container 400.

Figure 13A present details on a proximal end section of the container perforator 340. The proximal end of the container perforator 340 may be characterized as a corolla-shaped base equipped with crenulations, tabs, or retention sections 342, and that may be characterized as a stop for the container perforator 340. Preferably, the container perforator 340 comprises at least three such retention sections or tabs 342 that are spaced from one another. The container perforator 340 extends along a reference axis, and the tabs 342 extend away from the reference axis, proceeding in a direction of the base surface 329 of said spike assembly 300. A slit 347 is disposed between each adjacent pair of tabs 342. Stated another way, the tabs 342 are disposed about the circumference of the proximal end of the container perforator 340 and are disposed in spaced relation to one another. Any appropriate number of tabs 342 may be utilized, any appropriate spacing between adjacent pairs of tabs 342 may be utilized, or both. Generally, the tabs 342 are deflectable (e.g., elastically) structures.

The tabs 342 of the container perforator 340 may be characterized as being in the form of cantilevers. That is, each tab 342 has a free end 346 that is spaced from the base surface 329 of the spike assembly 300 and that corresponds with the proximal end of the container perforator 340. Each tab 342 may extend in diverging relation to the longitudinal axis of the spike assembly 300 proceeding in the direction of their corresponding free end. The tabs 342 may be curved proceeding along their respective length dimension. Stated another way, the tabs 342 may be characterized as defining a flared proximal end portion of the container perforator 340. In any case, there is a space 352 disposed between the tabs 342 and the perforation extension 380 (e.g., there is a space adjoining and radially inward of the tabs 342). The space 352 is an open space adjacent to and radially inward of each said tabs 342. A proximal section of each said tab 342 curves away from the reference axis proceeding to a corresponding said free end 346. Stated yet another way, at least proximal end sections of the tabs 342 and the perforator extension 380 may be disposed in radially-spaced relation to one another. Figure 13E shows the space 352 between the tabs 342 and the outer perimeter of the perforator extension 380 of the spike body 320, which allows elastic deformation of the crenulations 342 in the direction of the perforator extension 380.

An advantage associated with having the container perforator 340 include the tabs 342 is to allow a temporary elastic deformation of the container perforator 340 while the container perforator 340 passes into the septum 402 of the medical fluid container 400. When the container perforator 340 is in the material of the septum 402, it may be subjected to a radial force of between 10 and 30 N in the corolla-shaped area of the container perforator 340 (e.g., the area including the tabs 342) and, by elastic deformation, forces the displacement (e.g., the bending of the crenulations or tabs 342 radially inwards), which has the effect of reducing the effective outer diameter of the proximal end of the container perforator 340 (collectively defined by the tabs 342), and which may be such there is crenulation-to-crenulation contact by elimination of the slits 347 (e.g., the tabs 342 may be in contact with the perforator extension 380 at this time). This reduction in effective outer diameter of the proximal end of the container perforator 340 tends to reduce the perforation effort. When the tabs 342 of the perforator 340 open on the other side of the septum 402 - inside the medical container 400 - and become free of contact with the material of the septum 402, the tabs 342 should move back toward their undeformed state by the elasticity of the tabs 342 (which increases the effective outer diameter of the proximal end of the container perforator 340 but now within the interior of the medical fluid container 400). This increases the interference between the container perforator 340 and the septum 402 of the medical fluid container 400, making it more difficult to withdraw the container perforator 340 back out of the interior of the medical fluid container 400 through the septum 402. This interference may be characterized as the differential between the effective outer diameter of the tabs 342 in an "at rest" position (e.g., where an external force is not being exerted on the tabs 342 (e.g., Figure 13C), such as when disposed within the open, interior space of the medical fluid container 400), and the outer diameter tabs 342 when disposed against the outer perimeter of the perforator extension 380 of the spike body 320 by the engagement of the tabs 342 by the septum 402 as the spike assembly 300 is being installed on the container 400.

A corolla shape with elongated crenulations 342, shown in Figure 13A, may be characterized as providing a harpoon function for the container perforator 340. With the same flexibility, longer crenulations 342 allow an increase in the initial diameter and after opening a greater interference with the septum 402 of the medical container 400. The container perforator 340 may be configured such that the crenulations 342 on the interior-side of the septum 402 of the medical fluid container 400 will be perpendicular to this interior-side of the septum 402 if one attempts to withdraw the container perforator 340 out of the medical container 400 and without separating from a remainder of the container perforator 340. In any case and once the tabs 342 clear the interior side of the septum 402 of the medical container 400, the septum 402 will be engaged with the outer perimeter of the perforator extension 380 to provide a sufficient seal between the medical fluid container 400 and the spike assembly 300 during an injection.

Figures 13C and 13D present a variation of the container perforator 340 and are thus identified by reference numeral 340'. Corresponding components between the container perforator 340 and container perforator 340' are identified the same reference numerals, and the discussion presented herein regarding the container perforator 340 remains equally applicable to the container perforator 340' unless otherwise noted to the contrary. The container perforator 340' is configured to further reduce the forces required for the container perforator 340' to penetrate the septum 402 of the medical fluid container 400. Generally, a plurality of longitudinally-extending (e.g., elongate), circumferentially-spaced channels 360a (e.g., concave structures) are formed on an exterior surface or outer perimeter of the container perforator 340'. Each adjacent pair of channels 360a may be characterized as define a corresponding gadroon 360. In the illustrated embodiment, the width of the elongate channels 360a is progressively increased proceeding distally toward the distal tip 345 of the container perforator 340'. A width of each elongate channel 360a progressively decreases proceeding proximally away from an end closest to said distal tip 345 of said container perforator 340. As such, the width of the gadroons 360 is progressively decreased proceeding distally in the direction of the distal tip 345, thereby reducing the contact surfaces between the container perforator 340' and the septum 402 of the medical fluid container 400 (and thereby the amount of force required to direct the container perforator 340' through the septum 402 of the medical fluid container 400).

The gadroons 360 may extend at least generally from the base of the crenulations 342 to the location in the longitudinal dimension of the proximal end of where the passage 348 (air) intersects the exterior of the container perforator 340'. Any appropriate number of gadroons 360 may be utilized by the container perforator 340' and any appropriate spacing may be utilized between each adjacent pair of gadroons 360. In one embodiment, twelve gadroons 360 are utilized, with four gadroons 360 being disposed between each adjacent pair of windows 343 utilized by the container perforator 340' (three windows 343 in one embodiment of the container perforator 340'). The container perforator 340', via the channels 360a and corresponding gadroons 360, may reduce the contact surface with the septum 402 of the medical fluid container 400 by a factor of 2 compared to the container perforator 340.

Another variation of the container perforator 340 is illustrated in Figures 11 and 12 and is identified by reference numeral 340". Corresponding components between the container perforator 340 and the container perforator 340" are identified the same reference numerals, and the discussion presented herein regarding the container perforator 340 remains equally applicable to the container perforator 340" unless otherwise noted to the contrary. The container perforator 340' does not utilize the above-noted first transition section 341. Although the container perforator 340' does utilize a plurality of tabs 342' on its proximal end, these tab 342' do not define a "flared" proximal end section in the case of the container perforator 340'. However, the container perforator 340' is still configured to separate from the perforator extension 380 of the spike body 320 (or more generally from the spike body 320) in the above-noted manner (e.g., to reduce the potential for re-use of a spike assembly that utilizes the perforator 340".

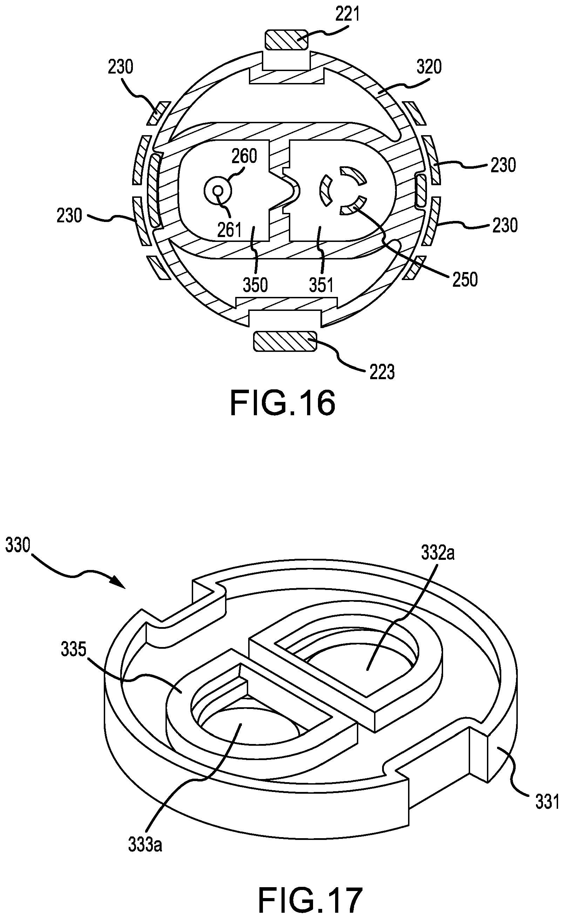

The flow of liquid out of the medical fluid container 400 and through the spike assembly 300, along with the flow of external air through the spike assembly 300 and into the medical container 400, will be discussed in relation to Figure 15A-15C and Figure 16. The spike assembly 300 again includes an interior chamber 350 (air) and an interior chamber 351 (liquid) that are each disposed in the spike body 320, more specifically within its base 370. The liquid chamber 351 is fluidly connected with the liquid passage 327, and the air chamber 350 is fluidly connected with the air passage 328, 348. A liquid in the medical fluid container 400 flows through the ports 343a in the windows 343 of the container perforator 340, into the one or more passages 327 in the perforator extension 380 of the spike body 320, and into the chamber 351 in the base 370 of the spike body 320. With the spike interface cover 200 being detachably connected to the spike assembly 300, the liquid passage 214 of the spike interface cover 200 is fluidly connected with the liquid chamber 351 and the air passage 261 of the spike interface cover 200 is fluidly connected with the air chamber 350. The perforator 250 for the liquid passage extends into said liquid chamber 351 and the perforator 260 for the air passage extends into said air chamber 350.Liquid in the chamber 351 of the spike assembly 300 flows into one or more passages 251 of the perforator 250 for the spike interface cover 200 (e.g., Figure 16), into the liquid passage 214 of the spike interface cover 200, into the container 100, and into the tubing 60. External air may flow into the spike interface cover 200 through the filter 240 (and filtering media 242), through the passage 261 of the perforator 260 for the spike interface cover 200, into the chamber 350 (e.g., Figure 16), into the passage 328 of the perforator extension 380, into the passage 348 of the container perforator 340, and into the medical fluid container 400.

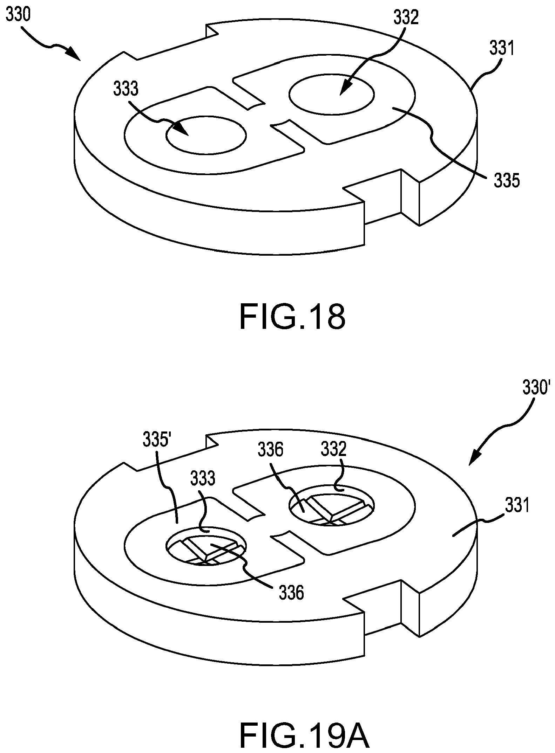

With the spike interface cover 200 being detachably connected to the spike assembly 300: 1) the perforator 250 (liquid) of the spike interface cover 200 extends through the port 333 of the interface 335 (incorporated by end cap 320) and into the chamber 351 of the spike assembly 300 (e.g., Figure 6); and 2) the perforator 260 (air) of the spike interface cover 200 extends through the port 332 of the interface 335 (incorporated by end cap 320) and into the chamber 350 of the spike assembly 300 (e.g., Figure 6). The interface 335 of the spike assembly 300 that includes the ports 332, 333 is illustrated in Figures 17 and 18 (along with one or more previously addressed figures, such as Figure 15A). The body 331 of the end cap 330 may be made of thermoplastic of ABS, MABS, PC or PA type, while the interface 335 of the end cap 330 may be made of SEBS, silicone or other soft materials with good deformability and low stress remanence, to seal with the functional areas of the chamber cover. The interface 335 can be over-molded to the body 331 on a lower or proximal end of the end cap 330 (corresponding with the lower or proximal end 334 of the spike assembly 330) or these components could be bonded together. Over-molding the interface 335 to the body 331 provides an advantage of providing an enhanced seal between these two components.

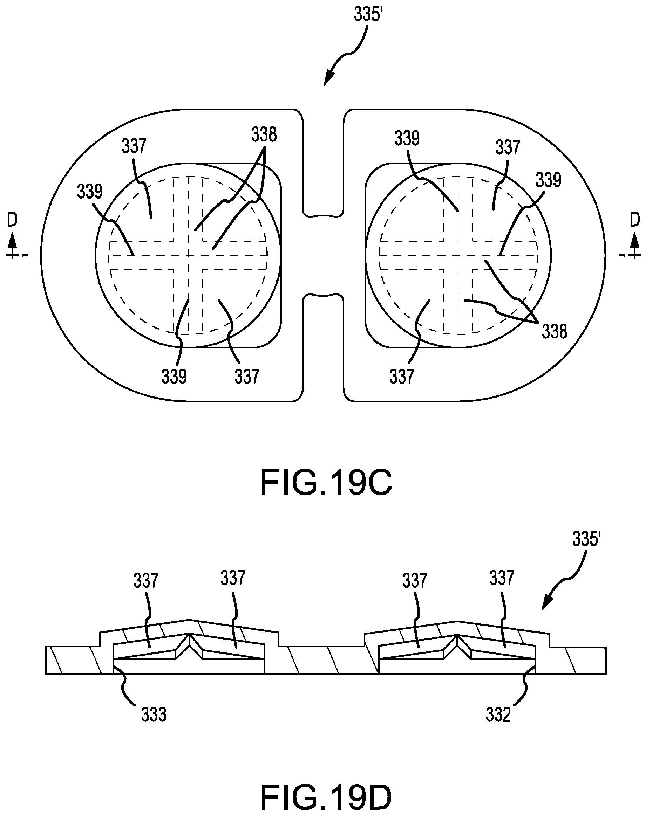

As illustrated in Figure 15A, a perforable wall 332a, 333a is disposed in and initially isolates each port 332, 333, respectively, from the corresponding chamber 350, 351, respectively. Each perforable wall 332a, 333a could be concave on a side that faces the corresponding perforator 260, 250, respectively, of the spike interface cover 200. Such a concave shape of the perforable walls 332a, 333a (e.g., see Figure 19D), as well as their respective thicknesses, are adapted in order to favour perforation during connection of the spike interface cover 200 to the spike assembly 300, and to favour re-closure after disconnection of the spike interface cover 200 from the spike assembly 300. When the spike interface cover 200 is detachably connected to the spike assembly 300 (where the perforator 250 (liquid) of the spike interface cover 200 extends into the port 333, through the corresponding perforable wall 333a, and into the chamber 351; where the perforator 260 (air) of the spike interface cover 200 extends into the port 332, through the corresponding perforable wall 332a, and into the chamber 350), the interface 335 of the spike assembly 300 seals against an outer perimeter of each of the perforators 250, 260 of the spike interface cover 200.

A variation of the end cap 330 (e.g., Figures 17-18) is illustrated in Figures 19A-19D and is identified by reference numeral 330'. Corresponding components between the end cap 330 and the end cap 330' are identified the same reference numerals, and the discussion presented herein regarding the end cap 330 remains equally applicable to the end cap 330' unless otherwise noted herein to the contrary. Generally, the end cap 330' uses a different configuration for the interface 335' (compared to the end cap 330), and that further improves upon the perforation and sealing functions with the creation of pre-cutting or break-out shapes that guide the opening of the material during perforation by the perforators 250, 260 of the spike interface cover 200.

The interface 335' includes the above-noted ports 332 (air), 333 (liquid) into which the perforator 260 and perforator 250, respectively, of the spike interface cover 200 may be directed to engage the corresponding perforable section 336 of the interface 335'. The perforable sections 336 of the interface 335' are each configured to separate in a predetermined manner (e.g., into segments 337; along predetermined separation lines 339 between each adjacent pair of segments 337) when engaged by the corresponding perforator 250, 260 of the spike interface cover 200. These segments 337 can be made, post-injection, reworked, with a cut in joined lips for example, using a blade, or in the injection process, with the creation of shapes in the injection mold. These segments 337 will generate thin thicknesses in the interface 335', these thin areas having the function of separation in a predetermined manner and guiding the opening of the interface 335'.

Figures 19A-D show the segments 337 being of a triangular configuration. Any appropriate number of segments 337 may be used per port 332, 332 (four in the illustrated embodiment). A chamfer 338 may be included on the sides of each segment 337 that adjoin another segment 337. These chamfers 338 may be characterized as providing the function of guiding the tearing or separation of adjacent pairs of segments 337 from one another, to guide the deformation, perforation, or "opening" of the interface 335' by the corresponding perforator 250, 260, or both. The chamfers 338 may be characterized as creating a corresponding thin-walled section of the interface 335' and in a manner that provides a desired seal of the interface 335' against the corresponding perforator 250, 260 by a predetermined and/or controlled separation of the interface 335' (the separation occurring along each thin-walled section - the separation lines 339).

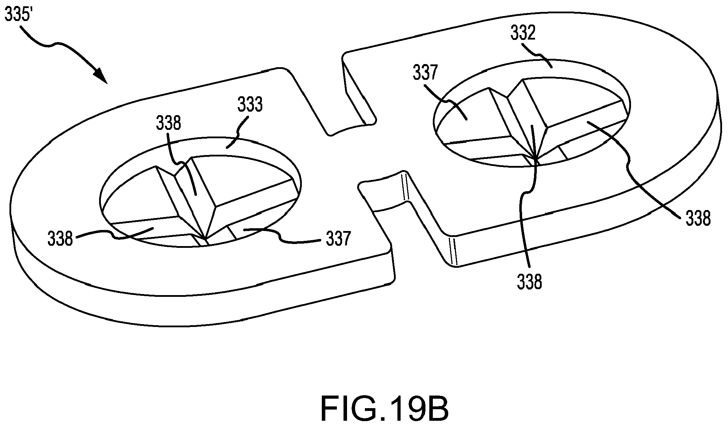

During perforation of the interface 335', the perforators 250, 260 of the spike interface cover 200 will contact at the center of the triangular segments 337 and in the center of the cross (the intersection of the two separation lines 339, per port 332, 333, shown in Figure 19C). Their pointed shape will perforate the thin material area and gradually tear along the profile until the right diameter is reached (Figures 19B-19D illustrate the interface 335' of the end cap 330', particularly showing thinner wall thicknesses in controlled tear areas (corresponding with the separation lines 339)). The slight tearing resistance of the material will open the opening sufficiently to allow the perforators 250, 260 to pass through while ensuring peripheral tightness and thus sealing. On disconnection of the spike interface cover 200 from a spike assembly utilizing the interface 335', the shapes will progressively withdraw from the part 335, the shape memory will make the slots progressively close again, and that should provide a sufficient seal for the corresponding medical fluid container 400.

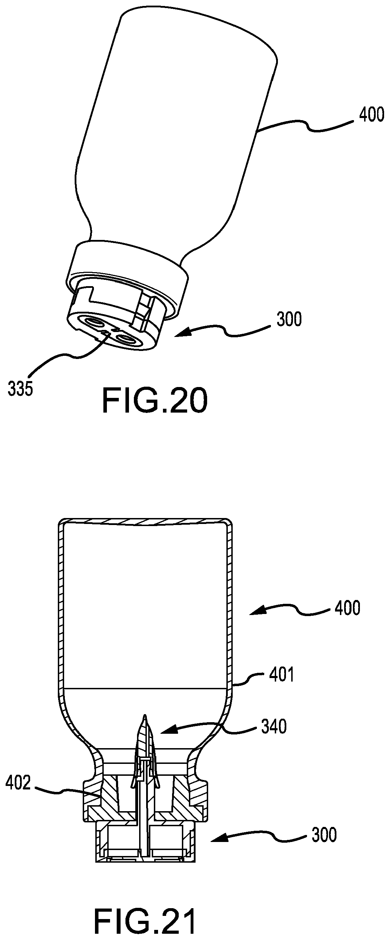

Figures 20 and 21 illustrate the spike assembly 300 installed on the medical fluid container 400, but with the spike interface cover 200 not being attached to the spike assembly 300. For instance, this may be the configuration of the medical container assembly 20 after a sufficient amount of the liquid in the medical fluid container 400 has been used (e.g., transported from the medical fluid container 400 via the spike assembly 300, the spike interface cover 200, the container 100, and the tubing 60), and when replacing the medical fluid container 400 (with the spike assembly 300 remaining installed thereon) for another medical fluid container 400 and where a new spike assembly 300 will be installed on this new medical fluid container 400 in accordance with the foregoing and where the multi-use spike interface cover 200 will be attached to this new spike assembly 300. In this regard and at the end of drawing liquid from the medical fluid container 400 (e.g., in the configuration of Figures 1 and 6), an operator may press on the actuating surfaces 220 of the spike interface cover 220, which pivots the clips 221, 223 about their corresponding hinge 222 to release the clips 221, 223 from the spike assembly 300 (by positioning the heads 224 of the clips 221, 223 "radially outward" of the corresponding locking surface 326 on an outer perimeter of the spike assembly 300). The spike assembly 300 and spike interface cover 200 are then uncoupled and the spike cover 200 may be moved axially away from the spike assembly 300 (again, with the spike assembly 300 remaining installed on the medical fluid container 400). On withdrawal, as soon as the perforators 250 and 260 of the spike interface cover 200 are withdrawn out of the interface 335 of the spike assembly 300, the holes previously created by the perforators 250, 260 in the interface 335 should close and seal the spike assembly 300 to reduce the potential for leakage of fluid from the medical fluid container 400 through the spike assembly 300. The medical fluid container 400, with the spike assembly 300 remaining installed thereon, may then be discarded reduced risk of discharge or residual product spillage from the medical fluid container 400.

As should be appreciated from the foregoing, the medical container adapter assemblies address one or more problems. One is that a number of different features may be incorporated by a container perforator of an adapter assembly to reduce the amount of force required to install a spike assembly on a medical fluid container. Another is that a number of different features may be incorporated by a spike assembly to reduce the chance of being able to re-use the same spike assembly on multiple medical fluid containers, which should reduce the potential for contamination. Another is that a spike assembly, a spike interface cover, or both may incorporate one or more features to reduce the potential for contamination prior to detachably connecting the spike interface cover with the spike assembly. Yet another is that a number of different features may be incorporated by a spike interface cover to allow the same spike interface cover to be re-used with multiple spike assemblies/medical fluid containers.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention. The embodiments described hereinabove are further intended to explain best modes known of practicing the invention and to enable others skilled in the art to utilize the invention in such, or other embodiments and with various modifications required by the particular application(s) or use(s) of the present invention. It is intended that the appended claims be construed to include alternative embodiments to the extent permitted by the prior art.

## Claims

1. A medical fluid container adapter assembly (40), comprising:
a first connector part (300) comprising a container perforator (340, 340', 340"), a first liquid passage (327) and a first air passage (328, 348), wherein said container perforator (340, 340', 340") comprises a distal tip (345) and an exterior, and wherein said first connector part (300) is connectable to a medical fluid container (400), wherein the first connector part is a single use connector part; and
a multi-use assembly (50) detachably connected to said first connector part (300), wherein said multi-use assembly (50) comprises a medical conduit interface (120) and a second connector part (200) comprising a second liquid passage (214) and a second air passage (261), and wherein said first liquid passage (327) and said first air passage (328) of said first connector part are fluidly connected with said second liquid passage and said second air passage, respectively, of said second connector part (200).

2. The medical fluid container adapter assembly of claim 1, wherein said first connector part (300) further comprises a first body (320) in turn comprising a first base distal surface (329), said container perforator (340, 340', 340") protrudes distally relative to said first base distal surface (329), and said container perforator (340, 340', 340") comprises said first liquid passage (327) and said first air passage (328).

3. The medical fluid container adapter assembly of claim 2, wherein said first connector part (300) is configured such that said container perforator (340, 340', 340") will separate from said first body (320) before said first body (320) will separate from the medical fluid container (400) when the medical fluid container adapter assembly (40) is in an installed configuration.

4. The medical fluid container adapter assembly of any of claims 2-3, wherein said first body (320) further comprises a base (370) and a perforator extension (380), said base (370) comprises said first base distal surface (329), said perforator extension (380) extends distally from said first base distal surface (329) and comprises said first liquid passage (327) and said first air passage (328), and said container perforator (340, 340', 340") is interconnected with said perforator extension (380).

5. The medical fluid container adapter assembly of any of claims 2-4, wherein said second connector part (200) comprises a second base distal surface (206), a perforator (250) for said second liquid passage (214), and a perforator (260) for said second air passage (261), wherein said perforator (250) for said second liquid passage protrudes distally from said second base distal surface (206), is fluidly connected with said second liquid passage (214), and is fluidly connected with said first liquid passage (327) of said first connector part (300), and wherein said perforator (260) for said second air passage protrudes distally from said second base distal surface (206), is fluidly connected with said second air passage (261), and is fluidly connected with said first air passage (328) of said first connector part (300).

6. The medical fluid container adapter assembly of claim 5, wherein said first body (320) comprises a liquid chamber (351) fluidly connected with said first liquid passage (327) and an air chamber (350) fluidly connected with said first air passage (328, 348), wherein said second liquid passage (214) of said second connector part (200) is fluidly connected with said liquid chamber (351) and said second air passage (261) of said second connector part (200) is fluidly connected with said air chamber (350), wherein said perforator (250) for said second liquid passage extends into said liquid chamber (351), and wherein said perforator (260) for said second air passage extends into said air chamber (350).

7. The medical fluid container adapter assembly of any of claims 5-6, wherein said second part (200) further comprises a plurality of upper wall segments (230) that are spaced from one another and that protrude distally from said second base distal surface (206) of said second connector part (200), said perforator (250) for said second liquid passage and said perforator (260) for said second air passage are located inwardly of an outer perimeter collectively defined by said plurality of upper wall segments (230), and a distal end of each of said perforator (250) for said second liquid passage and said perforator (260) for said second air passage are disposed closer to said second base distal surface (206) than a distal end of each of said plurality of upper wall segments (230).

8. The medical fluid container adapter assembly of any of claims 5-7, wherein said first body (320) comprises a proximal end (334) spaced from said first base distal surface (329) of said first body (320), said first connector part (300) comprises a first interface (335) disposed on said proximal end (334) of said first body (320) that interfaces with said second connector part (200), said first interface (335) is of a first hardness and said first body (320) is of a second hardness that is greater than said first hardness, said first interface (335) comprises a first perforable section (port 333) in a flow path to said first liquid passage (327) and a second perforable section (332) in a flow path to said first air passage (328, 348), said perforator (260) for said second liquid passage extends through said first perforable section (333) and into said liquid chamber (351), and said perforator (250) for said second air passage extends through said second perforable section (332) and into said air chamber (350).

9. The medical fluid container adapter assembly of any of claims 2-8, wherein said container perforator (340, 340', 340") extends along a first reference axis and comprises at least 3 retention sections (342) that are spaced from one another, that extend away from said first reference axis proceeding in a direction of said first base distal surface (329) of said first connector part (300), and that have a free end (346) that is spaced from said first base distal surface (329) of said first connector part, wherein an open space (352) is adjacent to and radially inward of each said retention section (342), and wherein a proximal section of each said retention member (342) curves away from said first reference axis proceeding to a corresponding said free end (346).

10. The medical fluid container adapter assembly of any of claims 2-9, wherein said first body (320) comprises first and second catches (321, 322) that are spaced from one another, wherein said second connector part (200) comprises a second body (210) and first and second clips (221, 223) that are movable relative to said second body (210) and that detachably engage said first and second catches (321, 322), respectively, to detachably connect said first connector part (300) and said second connector part (200), wherein said first and second catches (321, 322) are disposed on an outer perimeter of said first body (320).

11. The medical fluid container adapter assembly of claim 10, wherein said first body (320) comprises first and second channels (323, 324) disposed on said outer perimeter of said first body (320), wherein said first and second catches (321, 322) are disposed within said first and second channels (323, 324), respectively.

12. The medical fluid container adapter assembly of any of claims 10-11, wherein said first and second channels (323, 324) are configured to only accommodate a single relative orientation between said first connector part (300) and said second connector part (200) when detachably connected.

13. The medical fluid container adapter assembly of any of claims 1-12, wherein said container perforator (340, 340', 340") comprises a frusto-conical section (349) and a transition section (341), said frusto-conical section (349) is disposed between said distal tip (345) and said transition section (341), said transition section (341) is located between said frusto-conical section and said multi-use assembly (50), and said transition section (341) is a concave surface on said exterior of said container perforator (340) proceeding proximally relative to said frusto-conical section (349).

14. The medical fluid container adapter assembly of any of claims 1-13, wherein said container perforator (340) further comprises a plurality of elongate liquid inlet channels (343) that are spaced from one another on said exterior of said container perforator and that are concave, wherein each said elongate liquid inlet channel is fluidly connected with at least one said first liquid passage (327).

15. The medical fluid container adapter assembly of any of claims 1-14, further comprising a plurality of elongate channels (360a) that are spaced from one another on said exterior of said container perforator and that are concave, wherein a width of each elongate channel (360a) of said plurality of elongate channels progressively increases proceeding distally toward said distal tip (345) of said container perforator (340).
